# EUROPÄISCHE PATENTSCHRIFT

(11) **EP 1 827 271 B1**
(45) Veröffentlichungstag und Bekanntmachung des Hinweises auf die Patenterteilung: **02.12.2009**
(21) Anmeldenummer: 05815861.9
(22) Anmeldetag: 22.12.2005
(51) Int. Cl.: A61B 17/72, A61B 17/80

(54) **KNOCHENFIXATIONSVORRICHTUNG**
BONE FIXING DEVICE
DISPOSITIF DE FIXATION SUR LES OS

(30) Priorität: 23.12.2004 CH 21432004
(43) Veröffentlichungstag der Anmeldung: 05.09.2007
(73) Patentinhaber: Stäubli, Hans Ulrich, CH-3074 Muri (CH)
(72) Erfinder: Stäubli, Hans Ulrich, CH-3074 Muri (CH)
(74) Vertreter: Lusuardi, Werther
(86) Internationale Anmeldenummer: PCT/CH2005/000768
(87) Internationale Veröffentlichungsnummer: WO 2006/066440

(56) Entgegenhaltungen:
- EP-A- 0 689 800
- WO-A-02/080790
- US-A- 4 506 662
- US-A- 5 356 410
- US-A1- 2003 083 660

## Beschreibung

Die Erfindung bezieht sich auf eine Knochenfixationsvorrichtung gemäss dem Oberbegriff des Patentanspruchs 1.

Die erfindungsgemässe Knochenfixationsvorrichtung wird im folgenden speziell für die Anwendung an der Tibia beschrieben.
Zur Ausführung einer hohen Tibiakopfosteotomie sind mehrere Techniken bekannt: Prinzipiell werden sogenannte lateral zuklappende von medial aufklappenden Tibiakopfosteotomien unterschieden. Im englischen Sprachgebrauch wird von "high tibial osteotomy lateral closing wedge" und "medial opening wedge" gesprochen. Im Französischen spricht man von "ostéotomie du tibia d'ouverture interne" oder "d'addition interne". Neben diesen klassischen Osteotomieformen werden auch Halbschaftosteotomien mit Umkehrspaneinlage, domförmige Osteotomien und unterschiedliche Formen der Tuberositasosteotomien durchgeführt.
Zur Fixation der lateral zuklappenden Tibiakopfosteotomie werden gemäss Stand der Technik T-oder L-Platten sogenannte Rekonstruktionsplatten, Giebelplatten, Laschenplatten und zurechtgebogene Halbrohrplatten, TomoFix Platten sowie der Fixateur externe in unterschiedlichen Varianten angewendet Aaron Hofmann aus Salt Lake City Utah beschieb eine schräg verlaufende Schnittlehre sowie eine L-förmige Platte zur Fixation der hohen lateral zuklappenden Tibiakopfosteotomie unter dem Namen" Natural-Knee Family High Tibial Osteotomy System".
Zur Fixation der medial aufklappenden Tibiakopfosteotomie werden verschiedene Systeme verwendet: Das "Bone plate system for opening wedge proximal tibial osteotomy" gemäss US 5,620,448. Das "Bone plate system for proximal tibial osteotomy" gemäss US 5,749,875.
Lobenhoffer et de Simoni et Alex Staubli beschrieben in. "Open-Wedge High-Tiblal Osteotomy With Rigid Plate Fixation". Techniques in Knee Surgery 1(2):93-105, 2002 eine Knochenplatte, welche zur Stabilisierung der medial aufklappenden Tibiakopfosteotomie an die anteromediale Schlenbeinfläche der proximalen Tibia angelegt wird.
Rab publizierte 1988 die schräg ansteigende Tibiaosteotomie bei Kindern mit Blount's disease. "Oblique tibial osteotomy for Blount's disease", J.Pediatr. Orthop. 8:715-720, 1988.
Sanouillier Jean Louis und Rosa Thierry aus Frankreich publizierten eine verlängerte, proximal t-förmig ausgestaltete Osteotomieplatte mit auswechselbaren Keilen ("wedges") zur inneren Abstützung der medial aufklappenden Tibiakopfosteotomie gemäss FR 2,785,518.
Leone James et al. publizierten gemäss US Patent US 6,767,351 ein T-förmiges Plattensystem mit multidirektionalen Fixationsstiften.
Zur Fixation der medial aufklappenden Korrekturosteotomie werden auch externe Fixatoren angewandt. Von Ito et al. wurde 2001 ein verbesserter Verriegelungsnagel zur intramedullären Fixation beim osteoporotischen Knochen publiziert J Orthop Trauma 2001; 15:192-196

Zur endomedullären Fixation von Tibiaschaft- und proximalen Tibiafrakturen beim Erwachsenen wurde " Die Bündel-Nagelungen" 1961 durch K.H. Hackenthal, Berlin, Göttingen in einer Monographie beschrieben. Weitere Marknageltechniken sind: Die klassische Tibia-Nagelung in der Technik nach Küntscher, die konventionelle, aufgebohrte Tibia-Marknagelung, die unterschiedlichen Varianten der statischen und dynamischen Verriegelungsnagelungen gemäss Grosse/Kempf sowie gemäss den Empfehlungen der Zimmer GmbH (früher Sulzer Medica, Centerpulse) und die elastische Nagelung mittels Prevost- Métesiau- oder Nancy Nägeln bei Kindern. Merkmal dieser Marknagelungstechniken ist in der Regel ein transligamentärer Zugang durch Längsspaltung des Ligamentum Patellae unter Abschiebung des Hoffa'schen Fettgewebekörpers mit Restbeschwerden im Ansatzbereich des Streckapparates an der Tuberositas tibiae, heterotopen Ossifikationen und Schrumpfung des Kniescheibenbandes mit konsekutiver schmerzhafter Patella infera oder Patella baja (Tiefstand der Kniescheibe) und Beuge- sowie Streckausfall des Kniegelenkes. Selten stellt sich eine Sudeck'sche Dystrophie der Patella oder der Trochlea femoris bei Schrumpfung des Hoffa'schen Fettgewebekörpers oder des Ligamentum patellae als Komplikation ein.

Typischerweise werden die endomedullären Kraftträger, die in der sagittalen Ebene eine dorsal konvexe Krümmung im proximalen Teil des Tibiamarknagels aufweisen, durch einen transligamentären Zugang ins Schienbein oberhalb der Tuberositas tibiae eingeführt. Zur Verriegelung sind mehrere proximale, mittlere und distale Verriegelungsflxationsmittel bekannt.
In der deutschen Auslegeschrift DE 2.459.257 wurde ein distal scheibenförmig abgeflachter Nagel dargestellt, **dadurch gekennzeichnet, dass** eine Mehrzahl von Nagelenden übereinander zu liegen kommen.

Im deutschen Patent DE 7.519.604 wird ein Marknagel beschrieben, dessen Ende von der übrigen runden Nagelform abweicht zum Zwecke der Einleitung von Kräften und Drehmomenten.
In der EP 00669048 wurde ein elastischer Femurmarknagel offengelegt, mit einem verlängerten, zylindrischen, den halben Nagel miteinbeziehenden oberen Nagelende, das konkav ausgelegt ist, zur Aufnahme eines konvexen, T-förmigen ausgelegten Fixationsmittels das mit runden Aufnahmeöffnungen versehen ist.

Aus der EP-B 1 024 762 LEU ET AL. ist ein endomedullären Marknagel bekannt mit einem plattenähnlichen Aufsatz, der um eine annähernd senkrecht zur Nagelachse verlaufende Drehachse in eine beliebige Position zu einer auf dem Knochen aufliegenden Basisplatte rotiert und mittels Fixationselementen fixiert werden kann. Der koaxial zum Marknagel angeordnete Aufsatz ist somit ebenfalls endomedullär angeordnet und kann deshalb nur koaxial zum Marknagel um die gemeinsame Achse rotiert werden. Zudem fehlt bei der Vorrichtung von LEU ET AL. eine auf dem Knochen aufliegende Knochenplatte.

Weitere Beispiele für einen Marknagel mit dazugehöriger Fixierungsplatte finden sich in US 2003/083 660, EP-A 0 689 800 oder US 5,356,410.

Aus der US 5,603,715 KESSLER ist eine Vorrichtung mit einem hohlen Marknagel bekannt, welcher an seinem proximalen Ende einen orthogonal zur Nagelachse liegenden Zahnkranz aufweist. Die Vorrichtung umfasst weiter eine Knochenplatte mit einer senkrecht auf der Platte angeordneten Hülse, deren freies Ende einen zum Marknagel korrespondierenden Zahnkranz aufweist. Mittels einer Verbindungsschraube, welche durch die Hülse in den hohlen Marknagel durchgeführt wird, lässt sich die Knochenplatte in einer rotativ beliebigen Position zum Marknagel fixieren. Nachteilig bei dieser Vorrichtung ist die Beschränkung der Rotierbarkeit koaxial zur Nagellängsachse, so dass die Knochenplatte in den Bereich des Gelenkes kommt und dessen Funktion behindert. Des weiteren nachteilig ist, dass diese bekannte Platte proximal am Humerus auf die Weichteile das heisst auf die Rotatorenmanchette platziert dargestellt ist, mit der Gefahr der daraus entstehenden Bewegungseinschränkung des so versorgten Schultergelenkes mit konsekutiver Schultersteife. Eine Vorrichtung mit einem hohlen Marknagel, die im mittleren Teil geradlinig ausgelegt und rigide konstruiert ist lässt sich praktisch von distal her nicht in die enge Markhöhle eines Knochens mit enger Markhöhle wie derjenigen des dargestellten Humerusknochens einfügen. Aus diesem Grunde werden elastische Marknägel mit kleinem Durchmesser aus flexibler Titanlegierung zur Versorgung von Humerusschaftfrakturen verwendet (Prevost oder Nancy Nägel).

Hier will die Erfindung Abhilfe schaffen. Der Erfindung liegt die Aufgabe zugrunde, eine Knochenfixationsvorrichtung zu schaffen, mit welcher sowohl die knöchernen als auch die Weichteil-Strukturen weniger beeinträchtigt werden.

Die Erfindung löst die gestellte Aufgabe mit einer Knochenfixationsvorrichtung, welche die Merkmale des Anspruchs 1 aufweist.

Die durch die Erfindung erreichten Vorteile sind im wesentlichen darin zu sehen, dass dank der erfindungsgemässen Knochenfixationsvorrichtung:
a) eine extraartikuläre und extraligamentäre Platzierung (d.h. ausserhalb und sub-ligamentär unter das Ligamentum patellae aber nicht durch das Kniescheibenband hindurch) des implantates möglich ist, welche zur Schonung des Streckapparates und im besonderen des Ligamentum patellae sowie der Tuberositas tibiae führt;
b) das Kniegelenk und insbesondere die Tibia in leichter Beugestellung operiert werden kann, was die Fragmentreposition und Implantation der Fixationsvorrichtung wesentlich vereinfacht und die Weichteile schont;
c) keine wesentlichen Translationskräfte und Rotationsmomente auf die oberen, mittleren und unteren endomedullären Begrenzungen der Knochenmarkkanalanteile der Tibia ausgeübt werden;
d) der, den Knochen normalerweise bedeckende Weichteilmantel und insbesondere der Streckapparat inklusive Ligamentum Patellae und Hoffa'schem Fettgewebekörper im wesentlichen bei der Implantation/Explantation und bei der Fixation geschont werden;
e) eine Knieflexion von bis zu 110° sowie eine Spaltung des Ligamentum patellae vermieden werden kann.
f) Korrekturmöglichkeiten der Relativpositionen der durch die endomedullären und extramedullären Fixationsmittel festgelegten Knochenfragmentpositionen der gegeneinander verschiebbaren Knochenhauptfragmente in einem definierten Ausmass gegeneinander möglich sind;
g) erreichte Knochenfragment-Korrekturen gegeneinander in der korrigierten Position bis zur knöchernen Ausheilung und bis zum wiederhergerichteten Knochendurchbau fixierbar fest gehalten sind;
h) spätere Nachkorrekturen falls notwendig durch einfache Manipulationen erreichbar sind;
i) neurovaskuläre Komplikationen vermeidbar sind;
j) die periostale Knochenbruchheilung im meta-diaphysären Schaftbereich des Knochens geschont wird;
k) eine gegenüber dem zentralen Markraum exzentrisch gelegene breite weichteilschonend Knochenabstützung erreichbar ist;
l) eine Pluralität von Knochenfixationsmitteln der Knochenfixationsvorrichtung eine versatile Fixierbarkeit im epi-meta-diaphysären Knochenbereich garantiert;
m) gegenüber der EP-B1 024 762 LEU ET AL. keine Weichteil-Interferenz durch einen zentral axial ausgerichteten Nagelkopfteil resultiert; und
n) keine Schädigung des Streckapparates resultiert und damit der gefürchtete Patellatiefstand vermieden wird.

Im Gegensatz zu den konventionellen medial aufklappenden Tibiakopfosteotomien ist ein medialer Weichteilzugang auf Höhe des Schienbeinkopfes überflüssig. Die Pes anserinus Sehnen, das mediale Seitenband und das hintere Schrägband werden geschont, eine Nervenschädigung des Ramus infrapatellaris des Nervus saphenus wird vermieden. Die typische Vermehrung der Dorsalneigung des Schienbeinkopfes in der Sagittalebene ("Increased posterior slope"), sowie der mögliche Patellatiefstand wie wir sie bei der intraligamentären Aufklapposteotomie beobachten, entfallen. Ein medialer Weichteilzugang kann vermieden werden.
Im Gegensatz zu den lateral zuklappenden Tibiakopfosteotomien wird die Ablösung der anterolateralen Muskulatur von der anterolateralen Schienbeinkante überflüssig. Das Wadenbein muss nicht osteotomiert werden. Eine Schädigungsmöglichkeit des Nervus peroneus, der unter dem Fibulaköpfchen verläuft, entfällt; ein Kompartmentsyndrom der anterolateralen Muskelloge des Schienbeins ist sehr unwahrscheinlich. Die typische Schrägstellung der sog Gelenklinie des Kniegelenkes in der Frontalebene, wie wir dies nach lateraler Zuklapposteotomie beobachten, entfällt, ebenso die insuffizienz des lateralen Kapselbandapparates (Arcuatum-Komplex).

Dies erlaubt eine entsprechende Korrektur der Achsen, der Seitenverschiebung und der Länge der Tibia und stabilisiert die Fraktur- und Osteotomiefragmente der Tibia achsengerecht, sowie torsions-, und biegestabil, nach erfolgter Korrektur derselben, sicher bis zur knöchernen Ausheilung. Voraussetzung dafür sind ein adäquat hergestelltes, biomechanisch tragfähiges und winkelstabil flxierbares, materialgeprüftes endostales, endomedulläres Grundimplantat, bestehend aus geeigneten Ober-, Mittel-, und Unterteilen sowie ein dazu passendes Instrumenten- und Positionierungsset.
Die endostalen Fixationselemente der Oberteile, das intermediäre Verbindungselement, das distale, endomedulläre Fixationselement sowie die, die Tibia transfixierenden Fixationselemente sind anatomiegerecht skaliert ausgelegt variieren gemäss der äusseren und inneren Form des betroffenen Schienbeinkopfes und folgen der äusseren Geometrie und inneren Architektur des Schienbeins und dessen entsprechender, innerer Markraumhöhlen-Konfiguration.

Das intermediäre Zwischenelement ist seinerseits mittels Verdrehsicherungs-mechanismus sowie mittels geeignetem feinen Verzahnungssystem, vorzugsweise als Drehzapfen-, Drehplatten-Aequivalent ausgelegt, mit dem unteren endomedullären Fixationsmittel (endomedullärer Kraftträger EMKT) in dem Sinne verbunden, dass eine Achsenkorrektur, eine seitliche Verschiebung um typischerweise von 0 mm bis zu halber Schaftbreite sowie eine Längenkorrektur von 0 - 3 cm möglich sind. Das distale, an die innere Form des Medullarkanals der Tibia angepasste, endomedulläre Fixationselement lässt sich, nach erfolgter Korrektur, winkel-, torsions-, und achsenstabil mit den proximalen endostalen Fixationselementen verbinden. Das, die Fraktur- und Osteotomiezonen überbrückende, implantierbare Konstrukt, bestehend aus endostalen proximalen Fixationselementen, intermediärem Verbindungssystem und distalen, endomedullär fixierten Fixationselementen, bildet eine
stabile funktionelle Einheit, bis zur formalen knöchernen Ausheilung der Tibiafraktur/Tiblaosteotomie.
Die proximalen Fixationselemente werden im wesentlichen in der inneren Architektur des proximalen medialen, zentralen und lateralen Schienbeinkopfes endostal fixiert. Das daran angepasste, intermediäre Fixationselement ist mit kleinen Unebenheiten, Vorsprüngen vom darunterliegenden Periost abgesetzt und folgt mit seiner Passform der äusseren Geometrie des Schienbeinkopfes unter Respektierung der intakt belassenen Ansatzgeometrie und der Substanz des Ansatzes des Streckapparates (Ligamentum Patellae) an der Tuberositas tibiae sowie der Ansatzgeometrie des Tractus iliotibialis am Tuberculum von Gerdy. Das intermediäre Fixationselement kann auch zum Teil oder ganz in den Schienbeinkopf von ventral her versenkt sein. Das intermediäre Verbindungselement ist seinerseits mit dem distalen, endomedullären Kraftträger verbunden, und vorzugsweise als verdrehsicherbarer Mechanismus, typischerweise als dreidimensionales feinverzahntes Drehzapfen/Drehtellerkonzept ausgelegt, welches die stabile Verankerung der Fixationselemente garantiert. Eine weitere Verbindungsvariante ist als Scharnierkonzept ausgelegt.
Die distale Verankerung erfolgt mittels speziellen transossären Verbindungs- und/oder Verriegelungs-Fixationsmitteln, die gegebenenfalls durch elastische, im Grund-Implantat integrierte und eingebrachte Fixationsnägel ergänzt werden. Das orthopädisch/traumatologische, endostale, endomedulläre Grundimplantat ermöglicht im Verbund mit angepassten Instrumenten und dem dazu passenden Positionierungssystem, eine biomechanische adäquate Fixation der Fraktur- und Osteotomiefragmente über einen weichteilschonenden, minlinvasiven Zugang. Zur weiteren Weichteilschonung findet ein speziell adaptiertes, endomedulläres Knochendurchtrennungssystem Anwendung, das die Zerstörung des Periosts durch konventionell von aussen angeführte, vibrierende Sägeblätter vermeidet und damit der periostalen Knochenheilung dienlich ist. Die Assemblage und Desassemblage des endostalen, endomedullären Grundimplantates sind einfach ausgelegt. Die entstehenden Knochendefekte werden nach der weichteilschonenden Entfernung des orthopädisch/traumatologischen Grundimplantates offen belassen oder durch geeignete Knochenersatzmaterialien lückenlos aufgefüllt.

Die Knochenfixationsvorrichtung umfasst zweckmässigerweise eine Kompressions vorrichtung, mittels welcher die nagelseitige und die plattenseitige Klemmfläche gegeneinander pressbar sind. Die Kompressionsvorrichtung umfasst typischerweise ein Innengewinde in der Bohrung des Marknagels und eine durch die zentrale Öffnung der Knochenplatte hindurchführbare und in das Innengewinde der Bohrung schraubbare Klemmschraube.

Bei einer besonderen Ausführungsform weist der Marknagel zwei nagelseitige Klemmflächen auf. Diese Ausführung gestattet es, den proximalen Teil des Marknagels, wahlweise unter oder über der Knochenplatte zu fixieren. Im speziellen können die zwei nagelseitigen Klemmflächen im wesentlichen asymmetrisch exzentrisch zur Zentrallinie angeordnet sein.

Bei einer weiteren Ausführungsform ist die nagelseitige Klemmfläche eben ausgebildet und definiert eine Ebene B2, welche mit einer durch die Zentrallinie und den parallel zur Ebene B2 verlaufenden Durchmesser d des Marknagels definierten Ebene B1 einen Winkel alpha von 0° bis 30°, vorzugsweise von 10° bis 20° aufweist.
Alternativ kann eine zur Bohrungsachse orthogonale Ebene B2 mit einer durch die Zentrallinie und den parallel zur Ebene B2 verlaufenden Durchmesser d des Marknagels (1) definierten Ebene B1 einen Winkel alpha von 0° bis 30° , vorzugsweise von 10° bis 20° aufweisen. Bei einer weiteren Alternative kann eine zur Bohrungsachse orthogonale Ebene B2 parallel zu einer durch die Zentrallinie und den parallel zur Ebene B2 verlaufenden Durchmesser d des Marknagels definierten Ebene B1 verlaufen.

Bei einer besonderen Ausführungsform ist die nagelseitige Klemmfläche im Bereich der Bohrung des Marknagels gelegen.

Die plattenseitige Klemmfläche kann entweder nur an der Unterseite oder nur an der Oberseite der Knochenplatte ausgebildet sein oder auch sowohl an der Unterseite als auch an der Oberseite ausgebildet sein.
Bei einer weiteren Variante kann die plattenseitige Klemmfläche im Inneren der Knochenplatte ausgebildet sein und im wesentlichen parallel zu ihrer Unter- und Oberseite verlaufen. Bei dieser Ausführung wird die nagelseitige Klemmfläche in die U-förmig ausgebildete Knochenplatte eingeführt und dort - nach erfolgter Verklemmung - sandwichartig von der Knochenplatte umschlossen. Die U-förmig ausgebildete Knochenplatte kann dabei im Innern des U mit einer oder auch zwei Klemmflächen versehen sein; das gleiche gilt auch für den Marknagel, der ebenfalls eine oder zwei Klemmflächen aufweisen kann, so dass sich bei dieser Ausführung zwei Paare von Klemmflächen gegenüberstehen können, was die Klemmfläche und damit die Klemmwirkung verbessert.

Bei einer besonderen Ausführungsform kann die plattenseitige Klemmfläche im Bereich der Öffnung der Knochenplatte ausgebildet ist.

Die Zentrallinie kann die nagelseitige Klemmfläche unter einem durchschnittlichen Winkel von 1° - 20°, vorzugsweise von 2° -10° schneiden.

Bei einer weiteren Ausführungsform kann der proximale Endteil des Marknagels als separates, modulares Bauteil ausgebildet sein.
Bei einer weiteren Ausführungsform kann zwischen den zueinander korrespondierenden Klemmflächen ein Zwischenstück aus einem plastisch deformierbaren Material angeordnet sein. Dadurch lässt sich die sogenannte "fretting corrision" verhindern.

Der Marknagel kann bei einer speziellen Ausführungsform asymmetrisch, multiplanar gekrümmt sein. Bedingt durch die multiplanare, asymmetrische Krümmung lässt sich der Marknagel bei dieser Ausführung mühelos bei leicht gebeugtem Knie in die Markhöhle und in den Markraum einbringen.

Die zueinander korrespondierenden Klemmflächen des Marknagels und der Knochenplatte können je planar oder sphärisch oder zylindrisch ausgebildet sein. Die formkongruenten Fixationsflächen gestatten dabei eine gegenseitige Korrektur im Sinne der sechs Freiheitsgrade (6DOF= six degrees of freedom), d.h. um die drei Rotationsachsen: Flexion/Extension, Abduktion/Adduktion, Innenrotation/Aussenrotation und In den drei Translationsebenen: Anteriore Translation/posterioreTranslation, Mediale Translation /laterale Translation sowie in der Kompressionsebene/ Distraktionsebene; dies in einem erwünschten Ausmass aber gleichzeitig mit einer gewissen Begrenzung.

Die Klemmflächen weisen vorteilhafterweise eine dreidimensionale Strukturierung aufweisen, vorzugsweise in Form von Verzahnungen. Dies gestattet relative Winkelverschiebungen und relative Translationen zwischen Marknagel und Knochenplatte und eine gegenseitige form- und kraftschlüssig Fixation nach erfolgten Winkelverschiebungen und Translationen in gewünschter Position, z.B. mittels einer Klemmschraube. Die Verzahnungen können z.B. in Form von abgerundeten Windungen, Pyramidenspitzen oder von vielschichtigen Polygonen ausgebildet sein. Im weiteren können die Verzahnungsoberflächen gehärtet, beschichtet und/oder anodisch funkenanodisiert (im speziellen mittels anodischer Funkenanodisiering II) sein. Die Verzahnungsoberflächen können Im weiteren konkav oder konvex ausgelegt sein und/oder mit korrespondierend multiplanaren Flächen und wölbungskongruent ausgebildet sein.

Das distale Marknagelende kann vorteilhafterweise parabol symmetrisch abgerundet sein. Der Marknagel kann im weiteren mindestens neun planparallel angeordnete Längsrillen aufweisen, um damit eine Rotationsstabilisierung zu erreichen.

Das distale Ende des Marknagels kann im weiteren längs gespalten sein. Diese Ausbildung dient zur Vermeidung von Stresskonzentration am Nagelende bedingt durch den transligamentären Zugang und die konsekutive Fettköpervernarbung.

Die Knochenplatte kann eine konkave, gekrümmte Unterseite aufweist. Dadurch erfolgt eine optimale Anpassung an die Knochenoberfläche.

Die Knochenplatte kann auch eine konvexe, gekrümmt Oberseite aufweisen. Dies gestattet eine einfache Herstellung der gekrümmten Platte aus einer ebenen Platte.

Bei einer weiteren Ausführungsform kann die Unterseite und/oder die Oberseite sphärisch gekrümmt ausgebildet sein, wodurch sich ebenfalls eine einfache Herstellung ergibt.

Bei einer besonderen Ausführungsform weist die Unterseite der Knochenplatte eine Nute mit einem die plattenseitige Klemmfläche bildenden Nutenboden, einer ersten und einer zweiten Seitenwand auf. Die Nute weist zwischen der ersten und der zweiten Seitenwand eine minimale Breite b auf, welche eine mindestens teilweise Aufnahme des proximalen Endteils des Marknagels im Bereich der nagelseitigen Klemmfläche gestattet, so dass die nagelseitige Klemmfläche an der plattenseitigen Klemmfläche zur Anlage kommt. Die Nute gestattet eine seitliche Begrenzung der Bewegung des Marknagels relativ zur Knochenplatte während der Implantation, was für den Operateur eine Hilfestellung beim Zusammenstellen des Implantates darstellt. Das proximale Nagelende des Marknagels im Bereich der nagelseitigen Klemmfläche weist vorteilhafterweise eine Breite B > b auf. Der Vorteil dieser Ausführung liegt darin begründet, dass die seitliche Verschiebbarkeit durch Wahl der geeigneten Bauteile wählbar ist.

Bei einer weiteren Ausführungsform umfasst die Knochenfixationsvorrichtung mindestens ein eine Längsachse aufweisendes Knochenfixationselement (25). Das mindestens eine Knochenfixationselement kann vorteilhafterweise derart angeordnet sein, dass die Projektionen seiner Längsachse in die X,Z-Ebene, die X,Y-Ebene und in die Y,Z-Ebene eines dreidimensionalen Koordinatensystems mit den Achsen x,y,z relativ zur x-Achse (in der X,Y-Ebene), zur z-Achse (in der X,Z-Ebene) und zur z-Achse (in der Y,Z-Ebene) einen Winkel zwischen 0° und 60° einschliessen, wobei die z-Achse koaxial zur Zentrallinie (4) des Marknagels (1) im Bereich der nagelseitigen Klemmfläche (7) verläuft und die x-Achse orthogonal zur z-Achse und koaxial zu dem parallel zur Ebene (2) verlaufenden Durchmesser d des Marknagels (1) verläuft.

Bei einem Marknagel mit gekrümmter Zentrallinie kann die z-Achse durch die Tangente an die Zentrallinie (4) im Schnittpunkt der Zentrallinie (4) mit der die Drehachse bildenden Bohrungsachse (6) gebildet werden.

Die Oberfläche der Bauteile der Knochenfixationsvorrichtung, insbesondere des Marknagels, der Knochenplatte sowie allfälliger Knochenfixationselemente kann funkenanodisiert sein (vorzugsweise mittels anodischer Funkenanodisierung II) Dadurch erreicht man eine Erhöhung der mechanischen Festigkeit sowie eine mikrobizide Wirkung. Die Oberfläche der Bauteile kann auch mit einer - vorzugsweise Silberionen enthaltenden - mikrobiziden Beschichtung versehen sein.

Wie bereits erwähnt kann der Marknagel asymmetrisch ausgebildet sein. Dabei kann der Marknagel in einer ersten (frontalen oder latero-medialen) Ebene eine erste Krümmung und in einer, senkrecht zur ersten Ebene stehenden, zweiten (sagittalen oder antero-posterioren) Ebene eine zweite Krümmung aufweisen.

Die Knochenfixationsvorrichtung kann noch zusätzlich eines oder mehrere der folgenden Bauteile: eine Stoss-Schraube, eine Zug-Schraube, eine Kompressions-Schraube, eine Stellschraube, als Knochenfixationselemente umfassen.

Der Marknagel kann auch eine durchgehende Kannulierung aufweisen.

Bei einer weiteren Ausführungsform sind die Knochenfixationselemente als elastische Markraumdrähte ausgebildet.
Die Knochenfixationselemente können auch Hülsen umfassen, welche in Aufnahmen in der Knochenplatte eingepresst sind und deren Zentralbohrungen die Durchführung von Knochenschrauben gestatten.

Bei einer weiteren Ausführungsform ist die Knochenfixationsvorrichtung als Implantat zur endostalen und endomedullären Fixierung von Knochen, insbesondere von Tibiafrakturen, Tibiaosteotomien, Tibiakopffrakturen oder Tibiakopfosteotomien ausgebildet und umfasst dabei
A) mindestens ein Oberteil (23), welches im epiphysären, metaphysären und/oder diaphysären Bereich eines Knochens verankerbar ist;
B) mindestens ein Unterteil (21), welches im epiphysären, metaphysären und/oder diaphysären Bereich eines Knochens verankerbar ist; und
C) mindestens ein Mittelteil (22), welches zur Verbindung des mindestens einen Oberteils (23) mit dem mindestens einen Unterteil (21) geeignet ist und im epiphysären, metaphysären und/oder diaphysären Bereich eines Knochens verankerbar ist.

Das Oberteil kann mindestens ein, vorzugsweise mehrere Knochenfixationselemente umfassen. Das Oberteil kann auch mindestens ein laterales Knochenfixationselement (L), mindestens ein zentrales Knochenfixationselement (Z) und mindestens ein mediales Knochenfixationselement (M) umfassen. Das mindestens eine Knochenfixationselement kann eine Hülse (26) mit einer Zentralbohrung und eine durch die Zentralbohrung durchführbare Knochenschraube umfassen.

Das mindestens eine Knochenfixationselement kann eine Knochenschraube umfassen.

Das obere endostale laterale Fixationsmittel (L) im lateralen Schienbeinkopf kann in Bezug zur orthogonalen Ausrichtung in der Sagittal- und Koronarebene der individuellen Schienbeinkonfiguration folgen und geeigneterweise vom ventralen Eintrittsbereich des endostalen lateralen (L) Fixationsmittels, in einer Achsabweichung in der axialen Ebene im Sinne eines geeigneten Positionssektors, einen Winkel alpha in Bezug zur antero-posterioren Nullausrichtung von 0 bis 25 Grad nach zentral und von 0 bis 25 Grad nach lateral aufweisen, vorzugsweise als Winkel alpha von 0 bis 5 Grad nach zentral und lateral hin ausgelegt.

Das obere, endostale laterale Fixationsmittel (L) in Bezug zur sagittalen Neigung des Schienbeinplateaus und in Bezug zur anteroposterioren Ausrichtungsachse kann in der Sagittalebene in einem geeigneten Positionssektor, einem Winkel aeta von 0 bis 25 Grad nach cranial, sowie in einem Winkel aeta von 0 bis 45 Grad nach caudal ausgelegt sein, in Bezug zur sagittalen Seelenachse des endomedullären Kraftträgers in einem sagittalen Winkel theta von 0 bis 110 Grad vorzugsweise 0 bis 25 Grad ausgelegt sein, mit einem genügenden Abstand von mindestem 1 mm bis 3 mm zu den unmittelbar subchondralen, tibiaseitigen Bereichen des medialen und lateralen femoro-tibialen Gelenk-Kompartimentes sowie des Gelenkbereiches des proximalen Tibio-Fibulargelenkes sowie der Area interkondylaris anterior und der Area interkondylaris posterior der Schienbeinkopfepiphyse.

Das obere, laterale endostale Fixationsmittel (L) mit mindestens einem oberen epi-, meta-, diaphysären lateralen (L), zentralen (Z), medialen (M) Fixationsmittel des Oberteils, das Oberteil mit mindestens einem mittleren epi-, meta-, diaphysären Mittelteil, und das Mittelteil mit mindestens einem unteren diaphysären endostalen, exostalen, endomedullären Fixationsteil des Unterteils können in geeigneter. Art und Weise verbunden ausgelegt sein.

Das obere, endostale, laterale Fixationsmittel (L) mit mindestens einem endostalen, lateralen (L), zentralen (Z), medialen (M) endostalen, exostalen und endomedullären Fixationsmittel kann in geeigneter Weise stabil verbunden sein, unabhängig davon ob Achsen-, Längen-, und Winkelkorrekturen erfolgt sind.

Die oberen, endostalen lateralen (L), zentralen (Z), medialen (M), epi-, metaphysären endostalen Fixationsmittel können mit mindestens einem distalen endomedullär, exomedullär, endostal, exostal angebrachten Fixationsmittel (Unterteil des orthopädisch, traumatologlschen Grundimplantates) in geeigneter Weise achsen-, längen-, und winkelstabil verbunden sein.

Das obere, mindestens eine, endostale laterale epi-metaphysären Fixationsmittel (L) kann in geeigneter Art mit einem typischerweise ähnlich konfigurierten zentralen (Z) und medialen (M) epi-, metadiaphysären endostalen Fixationsmittel verbunden sein, das endomedulläre zentrale Fixationsmittel in Bezug zu einer anteroposterioren Orientierungs- oder Nullachse in der axialen Ebene als in einem Winkel beta von 0 bis 25 Grad nach zentral als in einem Winkel beta von 0 bis 45 Grad nach lateral ausgelegt, in der Sagittalebene in Bezug zu der anteroposterioren Nullachse als eine Winkelabweichung aeta von 0 bis 25 Grad in cranialer Richtung und eine Winkelabweichung aeta von 0 bis 25 Grad in caudaler Richtung ausgelegt.

Das endostale, laterale Fixationsmittel (L) und das endostale zentrale Fixationsmittel (Z) für das laterale (L), bzw. für das zentrale (Z) Fixationsmittel ausgelegt, können mit mindestens einem endostalen medialen (M) Fixationsmittel verbunden sein, das endostale, mediale Fixationsmittel (M) in Bezug zu einer in der Regel in 45 Grad schräg zur anteroposterioren Achse der Tibia orientierten Nullachse als in einem Winkel gamma von 0- 45 Grad nach ventral bis zu einem Winkel gamma von 0- 45 Grad nach dorsal ausgelegt, in der Sagittalebene in Bezug zu einer anteroposterioren Nullachse als eine Winkelabweichung aeta von 0 bis 25 Grad in cranialer Richtung und als eine. Winkelabweichung aeta von 0 bis 25 Grad in caudaler Richtung ausgelegt.

Das endostale laterale (L) und das endostale zentrale (Z) Fixationselement können für das laterale (L), bzw. für das zentrale (Z) endostale Fixationselement ausgelegt sein; das endostale mediale Fixationsmittel (M) in Bezug zu einer ventrodorsal orientierten Nullachse kann mit einen Winkel delta von 0 bis 20 Grad zur zentralen Sektorbegrenzung und einen Winkel delta von 0 bis 75 Grad bis zur ventralen Sektorbegrenzung ausgelegt sein.

Die endostalen Fixationselemente können aus mindestens zwei zentralen und mindestens zwei medialen, oder aus mindestens zwei lateralen und mindestens zwei medialen, je von ventral paraligamentär eingebrachten endostalen mehr oder weniger in der anteroposterioren Richtung orientierten Fixationsmitteln deren Nullachsen medial einen Winkel epsilon, lateral einen Winkel zeta bilden, ausgelegt sein.

Die zentralen (Z) und mediale (M) endostalen, epi-, meta-, diaphyären Fixationsmittel können mittels einer geeigneten Vorrichtung mit mindestens einem mittleren, intermediären (I) und mit mindestens einem unteren diaphysären endostalen- endomedullären Fixationsteil verbunden ausgelegt sein.

Die intermediären Fixationsvorrichtungen können, als Mittelteil ausgelegt, zwischen den einzelnen endostalen lateralen (L), endostalen zentralen (Z) und endostalen medialen (M) Fixationselementen des Oberteils und den endomedullären Fixationsteilen des Unterteils (des distalen, endomedullären Kraftträgers), gegeneinander typischerweise in einem Winkel von 0 bis 30 Grad abwinkelbar, zu jeder Seite hin von 0 mm bis um halbe Tibia-Schaftbreite verschiebbar, in der Länge um 0 cm bis 3 cm verschiebbar und nach erfolgter Korrektur in idealer Position wieder stabil miteinander verbindbar, ausgelegt sein.

Die endostalen epi-, meta-, diaphysären Fixationsmittel können aus Rohren, Teilen von Rohren, Zylindern, Teilen von Zylindern, Hülsen, Teilen von Hülsen, Stäben, Teilen von Stäben, Platten, Teilen von Platten, Laschen, Teilen von Laschen, Nägeln, Bolzen, Schrauben, Lamellen, elastischen Nägeln, T- und U-Profilen, mehrkantigen Elementen und Verriegelungsbolzen, Verriegelungsnägeln, winkelstabilen Schrauben, abwinkelbaren Schrauben, Bolzen oder anderen Fixationsvorrichtungen bestehen. Bei einer bevorzugten Ausführung können Führungshülsen und darin fixierte stabförmige Elemente im oberen Bereich der Tibia die mit einem intermediären Fixationsmittel (I), vorzugsweise als Basisplatte ausgelegt sowie mit vorzugsweise gebogen ausgelegten endomedullären (D) Fixationselementen im oberen, mittleren und distalen Bereich der Tibia befestigt sein.

Die intermediären Fixationsmittel (I), welche die endostalen epi-metaphysären Fixationsmittel mit den meta-, dia-, epiphysären endomedullären Fixationsmittel verbinden können aus, der äusseren und inneren Architektur des Schienbeinkopfes und dessen Oberfläche sowie aus den angrenzenden Weichteilen angepassten Elementen bestehen, die vorzugsweise als Abstützplatten mit gegenseitiger Verzahnung, als Drehzapfen-Drehplatten-Elemente, als Kugel-Pfannenelemente, als Gabel-Zapfen-Elemente, als Scharniervorrichtungen, als Drehteller - Drehzapfen-Elemente, als Kugel-Stabelemente ausgelegt sind.

Das intermediäre Fixationsmittel, das Mittelteil, als Knochenplatte oder Basisplatte (BP) ausgelegt, welches gegen die Vorderfläche des Schienbeinkopfs typischerweise mit Erhebungen und Vorsprüngen gegen den Knochen hin gerichtet, kann in einem Abstand von 0,1 mm bis 4 mm abgestützt sein und in geeigneter Weise die vordere Schienbeinbegrenzung bis maximal zum lateralen (äusseren) Rand des Schienbeinkopfes, seitlich und oberhalb der Tuberositas tibiae, hinter und neben dem Ligamentum patellae und dessen Ansatzzone im zentralen Schienbeinkopfbereich (Apophyse) bis hinüber zur medialen abgerundeten Schienbeinkopffläche bedecken und umgreifen und als Basisfixationsplatte für die geeigneten endostalen, endomedullären Fixationsmittel ausgelegt sein.

Die Basisplatte kann zu einem Drittel (in der Regel von 1 bis 33%), zu zwei Dritteln (in der Regel von 34 bis 66 %) oder zu mehr als zwei Dritteln (in der Regel von 67 bis 100% in den ventralen Bereich des Schienbeinkopfes versenkt sein, in Bezug zum Zentrum der Tuberositas tibiae in einem Radius von 5 mm bis 30 mm ausgelegt, mindestens 0,1 mm Distanz zum Ligamentum patellae Ansatz aufweisend, ausgelegt sein.

Die Basisplatte kann die paraligamentären lateralen und medialen Flächen des Schienbeins, und die zentral unter dem Ligamentum patellae liegende, vordere Schlenbeinkopffläche bedecken und das Einbringen von mindestens einem paraligamentär von ventral eingebrachten endostalen Fixationselement technisch ermöglichen.

Die Basisplatte kann auch ein in die Basisplatte direkt integriertes, im oberen lateralen (L), zentralen (Z) oder medialen (M) Bereich des Schienbeinkopfteils endostal fixiertes Fixationsmittel aufweisen.

Das endomedulläre Fixationselement kann in einer weiteren Ausführungsform um einen zentralen Fixationsmechanismus verdrehsicherbar fixiert sein, wobei die Fixationselemente der Basisplatte als schlitzförmige Aufnahmeöffnungen zur oberen und unteren Fixation des endomedullären Kraftträgers in der Basisplatte ausgelegt sind.

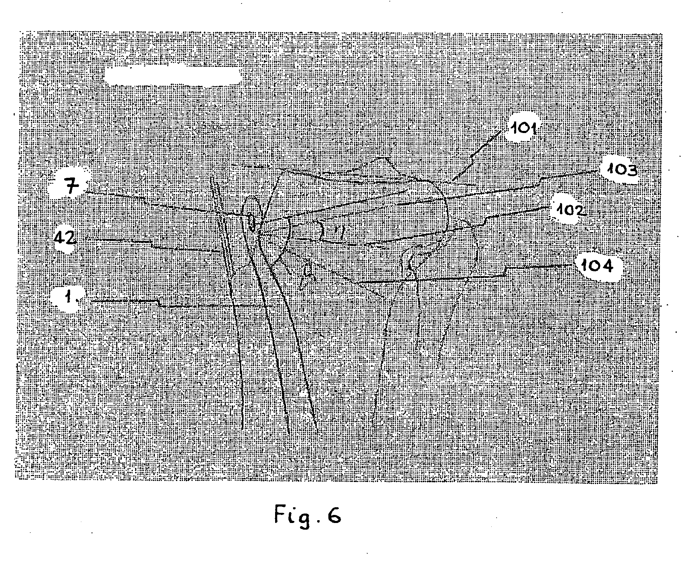

Die Basisplatte und der endomedulläre Fixationsteil können als fein verzahnte Drehzapfen, Drehtellermechanismen ausgelegt sein, welche die stabile Fixation des endomedullären Kraftträgers mit der Basisplatte gewährleisten unabhängig von einer eventuell notwendigen Korrektur der Fragmentstellung (siehe Figuren 6A,6B,6C,6D,6E).

Die oberen lateralen (L), zentralen (Z) und medialen (M) epi-meta diaphysären Fixationsmittel können in geeigneter Weise nach erforderlicher Verschiebung gegeneinander, miteinander winkelstabil und verschiebungssicher, verbindbar ausgelegt sein.
Das untere endomedulläre Fixationsmittel kann in geeigneter Weise mit dem intermediären Fixationsmittel der Basisplatte fest verbunden sein, vorzugsweise als Sektor einer Kugel ausgelegt mit einem zweiten kongruenten Kugelsektorelement in dem Sinne verbunden, dass Winkelkorrekturen möglich sind und mittels geeignetem Fixationsmodus fixiert gehalten.
Das endomedulläre Fixationsmittel, der endomedulläre Kraftträger (EMKT) zur Stabilisierung von Tibiafrakturen, Tibiaosteotomien ausgelegt, kann proximal mindestens eine in den EMKT integrierte Abstützplatte vorsehen, zur paraligamentären, retroligamentären endostalen Fixation in geeigneter Weise ausgelegt.
Das endomedulläre untere Fixationsmittel kann in seinem Verlauf gegen das distale Hauptfragment durch geeignete Fixationsmittel endomedullär, endostal, exostal und zur transossären Fixation ausgelegt sein. Der endomedulläre Kraftträger (EMFK) kann in geeigneter Weise mit dem distalen und proximalen tibialen Hauptfragment mittels geeigneter Fixationsvorrichtungen verbunden sein, und nach erfolgter relativer Positionsänderung der tibialen Hauptfragmente gegeneinander wieder in geeigneter Art winkel-, rotations- und teilbelastungsstabil ausgelegt sein.

Die erfindungsgemässe Knochenfixationsvorrichtung eignet sich insbesondere zur Durchführung eines Verfahrens zur endostalen und endomedullären Fixierung von Knochen, insbesondere von Tibiafrakturen, Tibiaosteotomien, Tibiakopffrakturen oder Tibiakopfosteotomien, wobei in mindestens drei verschiedenen Ebenen des zu behandelnden Knochens gezielte Knochenschwächungen zur plastischen Formbarkeit des Knochens erzeugt werden. Die Knochenschwächungen können mittels Bohren oder Sägen erzeugt werden. Gleichzeitig mit den Knochenschwächungen kann autologes, strukturiertes Knochenmaterial gewonnen werden.

Die Erfindung und Weiterbildungen der Erfindung werden im folgenden anhand der teilweise schematischen Darstellungen mehrerer Ausführungsbeispiele noch näher erläutert.

Es zeigen:
Fig. 1a eine schematische perspektivische Ansicht einer Ausführungsform der erfindungsgemässen Knochenfixationsvorrichtung;
Fig. 1b eine schematische Darstellung der zu den Klemmflächen korrespondierenden Ebenen;
Fig. 2 eine perspektivische Ansicht einer Ausführungsform des erfindungsgemässen Implantates zur Fixation von Knochen;
Fig. 3 eine perspektivische Ansicht einer weiteren Ausführungsform des erfindungsgemässen Implantates zur Fixation von Knochen;
Fig. 4A eine schematische Darstellung einer Ausführungsform der erfindungsgemässen Knochenplatte;
Fig. 4B eine schematische Darstellung einer weiteren Ausführungsform der erfindungsgemässen Knochenplatte;
Fig. 4C eine schematische Darstellung wiederum einer weiteren Ausführungsform der erfindungsgemässen Knochenplatte;
Fig. 5A - 5E schematische Darstellungen der Fixationssektoren der endostalen Fixationselemente in der axialen Ebene des Schienbeinkopfes;
Fig. 6 eine schematische Darstellung der Fixationssektoren der endostalen Fixationselemente in der sagittalen Ebene;
Fig. 7A - 7E schematische perspektivische Darstellungen von verschiedenen Ausführungsformen des Fixationsmechanismus zwischen der Knochenplatte und dem Marknagel der erfindungsgemässen Knochenfixationsvorrichtung; und
Fig. 8 eine Explosionsdarstellung einer weiteren Ausführungsform des erfindungsgemässen Implantates.

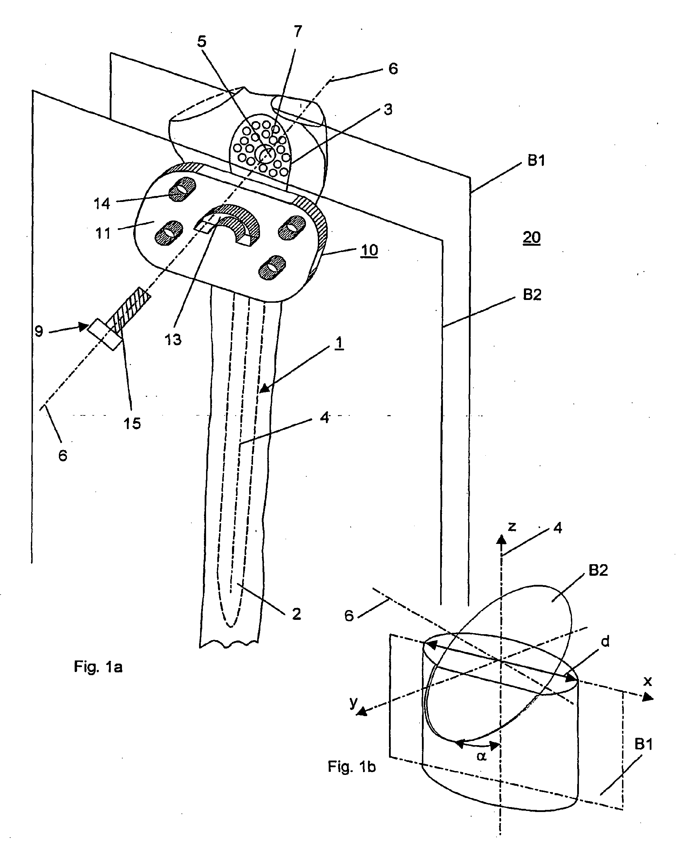

In Fig. 1a ist eine Ausführungsform der Knochenfixationsvorrichtung mit einem Marknagel 1 einer Knochenplatte 10 und einem Kompressionsmechanismus 9 zur Fixierung der Knochenplatte 10 am Marknagel 1. Der Marknagel 1 umfasst ein distales Endteil 2, ein proximales Endteil 3, eine Zentrallinie 4 und eine im proximalen Endteil 3 quer zur Zentrallinie 4 verlaufende, als Lager für eine Drehachse geeignete Bohrung 5 mit der Bohrungsachse 6. Die Knochenplatte 10 weist eine Oberseite 11 und eine für den Knochenkontakt bestimmte Unterseite 12 auf und umfasst eine im zentralen Teil der Knochenplatte 10 untergebrachte, die Oberseite 11 mit der Unterseite 12 verbindende, durchgehende, zentrale Öffnung 13 und mehrere, peripher um die zentrale Öffnung 13 angeordnete, ebenfalls durchgehende Plattenlöcher 14.

Ferner ist am proximalen Endteil 3 des Marknagels 1 eine nagelseitige Klemmfläche 7 ausgebildet, und die Knochenplatte 10 weist eine zur nagelseitigen Klemmfläche 7 korrespondierende plattenseitige Klemmfläche 16 auf, wobei die beiden zueinander korrespondierenden Klemmflächen 7,16 im ungeklemmten Zustand, d.h. bei gelöster Kompressionsvorrichtung 9 gegeneinander verschiebbar und rotierbar sind und im geklemmten Zustand gegeneinander fixiert sind. In der in Fig. 1a dargestellten Ausführungsform sind die Klemmflächen 7,16 planar ausgebildet. Die nagelseitige Klemmfläche 7 ist im Bereich der Bohrung 5 gelegen während die plattenseitige Klemmfläche 16 durch die Unterseite 12 definiert ist.

Die beiden zueinander korrespondierenden Klemmflächen 7,16 sind mittels einer durch die zentrale Öffnung 13 und die Bohrung 5 hindurchführbaren Klemmschraube 15 gegeneinander verklemmbar, wobei die Klemmschraube 20 im Knochen oder in der Bohrung mittels einer Gewindeverbindung verankerbar ist. Die Klemmflächen 7,16 weisen als dreidimensionale Strukturierung je eine Verzahnungen auf.

Die planare, nagelseitige Klemmfläche 7 liegt in einer Ebene B2, welche orthogonal zur Bohrungsachse 6 ist (Fig. 1b). Ferner wird durch die Zentrallinie 4 - bei gekrümmter Zentrallinie 4 durch die Tangente im Schnittpunkt mit der Bohrungsachse 6 - und dem parallel zur Ebene B2 verlaufenden Durchmesser d des Marknagels 1 eine zweite Ebene B1 aufgespannt. Je nach Ausführungsform der nagelseitigen und der plattenseitigen Klemmfläche 7, 16 schneiden sich die zwei Ebenen B1;B2 und schliessen einen Winkel alpha ein, oder die zwei Ebenen B1;B2 sind parallel oder fallen zusammen. In der in Fig. 1a dargestellten Ausführungsform sind die zwei Ebenen B1 und B2 parallel und weisen einen Abstand a zueinander auf.

Ferner ist in Fig. 1b ein dreidimensionales Koordinatensystem dargestellt, dessen z-Achse koaxial zur Zentrallinie 4 (Fig. 1 a) des Marknagels 1 im Bereich der nagelseitigen Klemmfläche 7, respektive bei einem Marknagel 1 mit gekrümmter Zentrallinie 4 tangential durch den Schnittpunkt der Zentrallinie 4 mit der die Drehachse bildenden Bohrungsachse 6 verläuft. Die x-Achse verläuft orthogonal zur z-Achse und koaxial zu dem parallel zu Ebene B2 verlaufenden Durchmesser d des Marknagels 1.

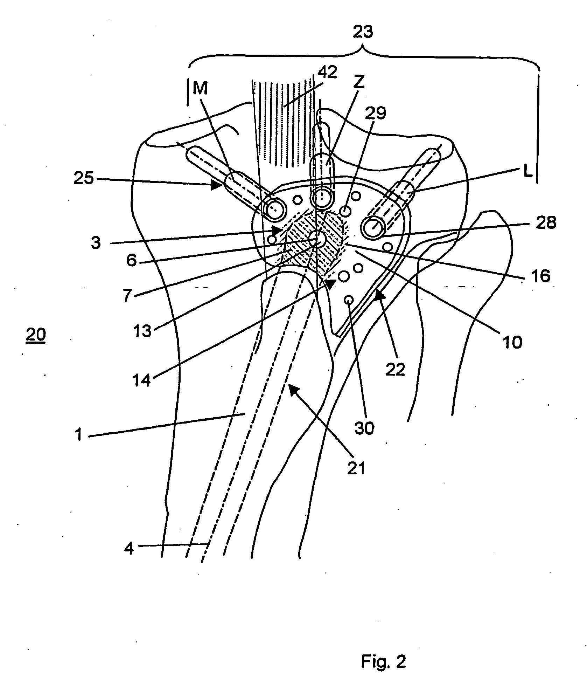

Das in Fig. 2 dargestellte System zur endostalen und endomedullären Fixation von Knochen umfasst ein Orthopädisch/traumatologisches Grundimplantat 20 im nachfolgenden OTGI genannt, welches ein als Marknagel 1 ausgebildetes Unterteil 21, ein als Knochenplatte 10 oder Basisplatte (BP) ausgebildetes Mittelteil 22 und ein aus mehreren Knochenfixationselementen 25 bestehendes Oberteil 23 beinhaltet: In der hier dargestellten Ausführungsform sind drei Knochenfixationselemente 25 vorgesehen, /wovon das Erste lateral (L), das Zweite zentral (Z) und das Dritte medial (M) angeordnet ist.

Die Knochenfixationselemente 25 sind derart angeordnet, dass die Projektionen ihrer Längsachsen 24_{L}; 24_{Z} und 24_{M} in die X,Z-Ebene, die X,Y-Ebene und in die Y,Z-Ebene (Fig. 1 b) relativ zur x-Achse (in der X,Y-Ebene), zur z-Achse (in der X,Z-Ebene) und zur z-Achse (in der Y,Z-Ebene) winklig angeordnet sind.

Der Marknagel 1 und die Knochenplatte 10 sind an die äussere Form und an die innnere Architektur des oberen Schienbeinteiles (an die sogenannte Epiphyse, Metaphyse, Apophyse und Diaphyse der proximalen Tibia und an die endomedulläre Form (Markraumform) des Schienbeins angepasst und folgen in skalierter Ausführung der inneren und äusseren Form der Tibia unter Intaktbelassen des Ligamentum patellae 42, der anterolateralen Muskelloge, des distalen Ansatzes der Pes Anserinus-Sehne, zur stabilen endostalen, exostalen, endomedullären Fixation von Tibiakopffrakturen, Tibiafrakturen, Tibiakopfosteotomien, Tibiaosteotomien.

Der Marknagel 1 und die Knochenplatte 10 kommen typischerweise hinter (dorsal) und unter das Ligamentum patellae (subligamentär) zu liegen. Durch die subligamentäre Lage der Knochenplatte 10 oder Basisplatte und des Marknagels 1 wird das Ligamentum patellae 42 und der Hoffa'sche Fettgewebekörper geschont, ein sekundärer Patellatiefstand wie beim transligamentären Zugang lässt sich somit mit grosser Wahrscheinlichkeit vermeiden.

Die in Fig. 2 dargestellte Ausführungsform des Implantates 20 zeigt ein orthopädisch, traumatologisches Grundimplantat (OTGI) bestehend aus Ober-, Mittel-, und Unterteilen 23,22,21 und ist dadurch charakterisiert, dass das Oberteil 23 aus mehreren endostale Fixationselementen 25 besteht, welche hier ein laterales (L), zentrales (Z) und mediales (M) endostale Fixationselement 25 umfassen, das Mittelteil 22, auch Intermedär-Teil genannt, aus einer anatomiegerechten, an die Weichteile adaptierten Knochenplatte 10 oder Basisplatte besteht, welche mit Plattenlöchern 14 als Aufnahmevorrichtungen 28 für die endostalen Fixationselemente 25, als Zusatzaufnahmevorrichtungen 29 für Zusatzfixationselemente beispielsweise Knochenschrauben und als Aussparungen 30 für die Weichteiladaptation versehen ist. Ferner umfasst die Knochenplatte 10 eine plattenseitige Klemmfläche 16, d.h. hier eine geeignet ausgelegte, feinstrukturierte Oberfläche, welche vorzugsweise als feinverzahnte Fixationsvorrichtung ausgelegt ist und in eine die nagelseitige Klemmfläche 7 bildende, reziprok ausgelegte Fixationsvorrichtung eingreift, so dass die plattenseitige Klemmfläche 16 und die nagelseitige Klemmfläche 7 rotativ formschlüssig miteinander verbunden sind. Die zuletzt genannte Fixationsvorrichtung ist mit dem oberen Teil des gekrümmten endomedullären Kraftträgers, respektive Marknagels 1 vorzugsweise winkelstabil verbunden, wobei diese Fixationsvorrichtungen, d.h. die nagelseitige Klemmfläche 7 und die plattenseitige Klemmfläche 16 vorzugsweise um eine mit der Bohrungsachse 6 (senkrecht zur Zeichenebene) zusammenfallende Drehachse, die In einem Winkel alpha von 65° - 115°, vorzugsweise 90 ° zur Schaftachse respektive Zentrallinie 4 des endomedullären Kraftträgers ausgelegt ist, relativ zueinander rotierbar und winkelstabil fixierbar sind.

Das Implantat 20 (OTGI) zeigt typischerweise endostale proximal lokalisierte Fixationselemente 25, die als Oberteil 23 ausgeformt sind und mit der retro- und paraligamentär gelegenen Knochenplatte 10 oder Basisplatte, die vorwiegend exostal fixiert wird, verbunden sind.
Das mittlere Teil, die Basisplatte, ist ihrerseits mit einem endomedullären Kraftträger, bzw. Marknagel 1 in geeigneter Weise so verbunden, so dass relative Achsenkorrekturen, seitliche Lageabweichungen und seitliche Verschiebungen sowie gegebenenfalls Winkelkorrekturen zwischen den proximalen und distalen Fraktur- und Osteotomie-Fragmenten möglich sind. Eine vorzugsweise als feinverzahnter Drehzapfen/Drehteller ausgelegter Fixationsmechanismus 8 (vgl. Fig. 1) erlaubt die Korrekturmöglichkeiten und garantiert die Stabilität nach erfolgter Korrektur bis zur knöchernen Konsolidierung.

Der Abstand der Knochenfläche zur lateralen, kranialen und medialen Begrenzung der Tuberositas tibiae ist bewusst gewählt, um eine gewisse Verschiebbarkeit der Knochenplatte 10 gegenüber der Tuberositas tibiae zu ermöglichen.

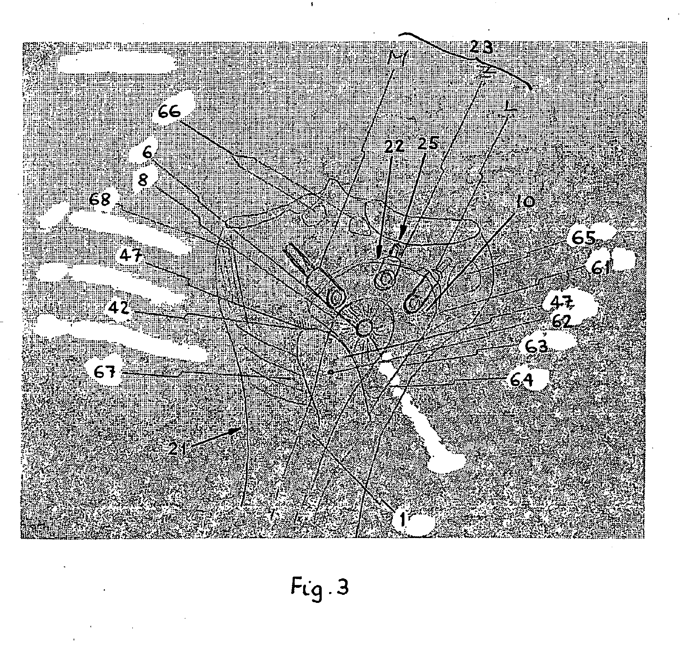

In Fig. 3 ist eine weitere Ausführungsform der Knochenplatte 10 oder Basisplatte (2) des Implantates 20 (OTGI) dargestellt. Die Knochenplatte 10 ist das Mittelteil 22, welches die endostalen proximalen lateralen (L), zentralen (Z) und medialen (M) Fixationselemente 25 des Oberteils 23 mit dem distalen Unterteil 21, dem endomedullären Kraftträger, d.h. dem Marknagel 1 via feinverzahnten Fixationsmechanismus 8 winkelstabil verbindet.

Wie in Fig. 3 dargestellt folgt die knochennahe Form der Knochenplatte 10 oder Basisplatte der äusseren Kontur des anterolateralen Schienbeinkopfs 61. Die Knochenplatte 10 oder Basisplatte bedeckt vorzugsweise die ventrale Fläche des anterolateralen Schienbeinkopfs 61 in einem Radius von 5 - 30mm; vom Zentrum 62 der Tuberositas tibiae 47, dem Ansatz des Ligamentum patellae 42, aus gemessen, bis zur lateralen Schienbeinkopfbegrenzung 63, wo sich das Ansatzareal der Faszie der anterolateralen Muskulatur 64 der Tibia befindet. Gegen oben seitlich wird dieses potentielle Platzierungsareal der Basisplatte durch das Tuberculum von Gerdy 65 begrenzt (tibiales Ansatzareal des Tractus iliotibialis). Gegen oben hin wird das Platzierungsareal der Basisplatte durch das Ligamentum coronarium, die Meniskusvorderhörner 66 und den Hoffa'schen Fettgewebekörper, medial unten durch den Ansatz der Pes Anserinus Sehnen 67, unten durch die proximale Begrenzung der Tuberositas tibiae 47 medial durch das mediale Seitenband 68 und ventral durch das Ligamentum patellae 42 begrenzt. Der in die Basisplatte integrierte, feinverzahnte Fixationsmechanismus 8 ist vorzugsweise flach ausgelegt, mit einer um eine zur Bohrungsachse 6 koaxialen Drehvorrichtung 69 ausgestaltet, welche eine Relativverschiebung der ineinandergreifenden feinverzahnten Fixationsvorrichtungen der Knochenplatte 10 oder Basisplatte und des endomedullären Kraftträgers, bzw. Marknagels 1 erlaubt und nach erforderlicher Korrektur in geeigneter Weise winkelstabil sichert.

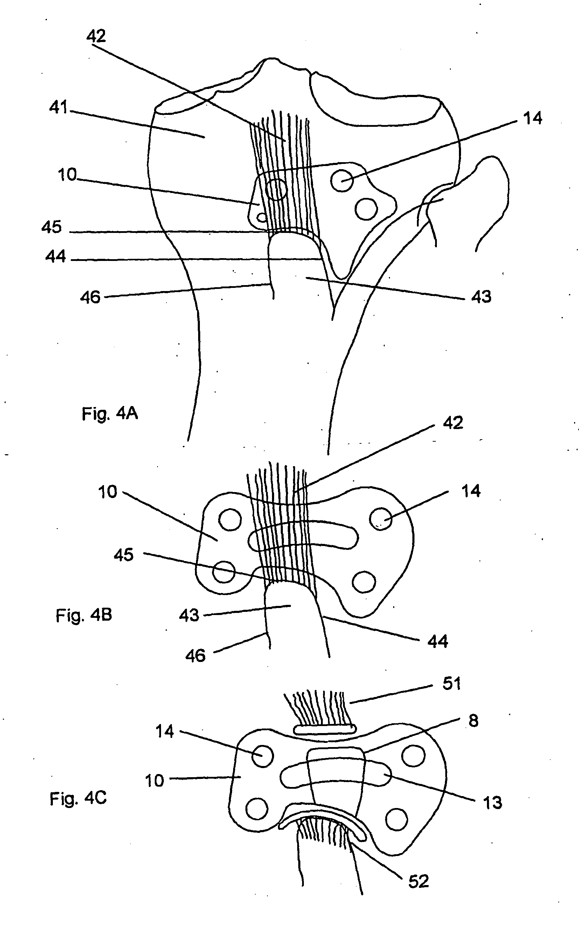

Fig. 4A - 4C zeigen eine Ausführungsform der Knochenplatte 10 oder Basisplatte. Die Knochenplatte 10 bedeckt im wesentlichen die von direkten Weichteilansätzen freien Areale der proximalen Epi-, Metaphyse des Schienbeinkopfes 41 und liegt hinter dem Ligamentum patellae 42 das intakt belassen wird. Die Knochenplatte 10 umgreift ansatzschonend die laterale 44, craniale 45 und mediale 46 Begrenzung des knöchernen Ansatzes 43 des Ligamentum patellae 42 an der Tuberositas tibiae, eine gewisse Distanz zu derselben einhaltend.
Die Kontur der Knochenplatte 10 folgt im wesentlichen der Ansatzgeometrie der Tuberositas tibiae 47 und ist vorzugsweise an die laterale 44 und craniale 45 Begrenzung der Tuberositas tibiae 47 mit Aufnahmeöffnungen zur Fixation der proximalen endostalen Fixationselemente ausgelegt (Fig. 3A).

Die Knochenplatte 10 ist in einer weiteren Ausführung so ausgelegt, dass sie im wesentlichen die laterale 44, craniale 45 und mediale 46 Begrenzung der Tuberositas tibiae 47 umgreift, so dass vorzugsweise die endostalen Fixationselemente 25 paraligamentär, das heisst das Ligamentum patellae 42 schonend, eingebracht sind (Fig. 4B).
Die Knochenplatte 10 ist in einer weiteren Ausführungsform so ausgelegt, dass die endostalen Fixationselemente 25 in geeigneter Weise nahe der Peripherie angebracht sind und im zentralen Bereich der Knochenplatte 10 eine zentrale Öffnung 13 angeordnet ist, so dass die Knochenplatte 10 in geeigneter Weise für einen verschiebbaren Fixationsmechanismus 8 ausgelegt ist (Fig. 4C).
In Fig. 4C ist das Ligamentum patellae 42, unter das die Knochenplatte 10 positioniert ist, zeichnerisch als proximal 51 und distal 52 durchtrennt dargestellt.

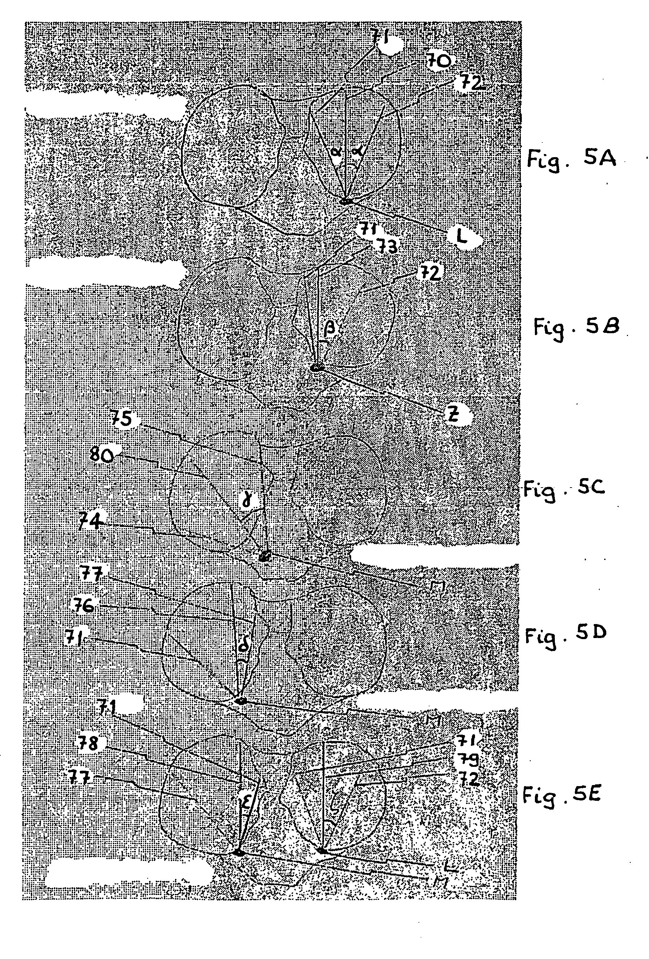

In Fig. 5A - 5E sind die Fixationssektoren der endostalen Fixationselemente 25 (Fig. 1) in der axialen Ebene dargestellt: Die endostalen lateralen (L), zentralen (Z), medialen (M) cranial oder caudal gelegenen endostalen Fixationselemente 25 sind vorzugsweise als Hülsen-Stabelemente, beispielsweise als in die Plattenlöcher 14 (Fig. 1) einführbare Hülsen mit Pins, welche durch deren Zentralbohrungen durchführbar sind, ausgelegt und sind in der axialen Ebene im wesentlichen von ventral nach dorsal eingebracht.

Das laterale endostale Fixationselement (L) ist im subchondralen Bereich des lateralen femorotibialen Kompartimentes so eingebracht, dass von der ventralen Eintrittstelle aus gemessen, im Vergleich zu der orthogonalen Ausrichtung in der antero-posterioren Richtung der Null- Position 70 aus, ein Positionssektor in einem Winkel alpha von plus minus 25 Grad von zentral 71 nach lateral 72 hin, vorzugsweise mit einer Abweichung von plus minus 5 Grad definiert ist (Fig. 5A).

Das zentrale endostale Fixationselement (Z) ist im zentraler gelegenen Bereich des lateralen femorotibalen Kompartimentes in geeigneter Weise in ventro-dorsaler Richtung subchondral in Bezug zur Nullpositionsachse 73 eingebracht mit einer möglichen Positionsabweichung einem Winkel beta nach zentral 71 von 20 Grad und nach lateral,72 von 35 Grad entsprechend (Fig. 5B).

Das mediale endostale Fixationselement (M) ist vorzugsweise von anterolateral her subligamentär im Vergleich zu einer schräg von anterolateral nach posteromedial orientierten Null-Achse 80 mit einer Winkelabweichung gamma von 35 Grad nach ventral 74 und von 45 Grad nach dorsal-zentral 75 in den subchondralen Bereich des medialen femorotibialen Kompartimentes eingebracht (Fig. 5C).

In einer weiteren Ausführungsvariante ist das mediale endostale Fixationselement (M) von ventral paraligamentär her im subchondralen Bereich des medialen femorotibialen Kompartimentes so eingebracht, dass von der ventro-dorsal ausgerichteten Nullposition 76 aus, eine Winkelabweichung delta von 45 Grad nach medial 77 bis zu 20 Grad nach zentral 71 definiert ist, vorzugsweise plus minus 5 Grad (Fig. 5D).

In einer weiteren Ausführungvariante sind mindestens zwei endostale laterale (L) und zwei endostale mediale (M) Fixationselemente je von ventral lateral und von ventral medial paraligamentär vorzugsweise in der antero-posterioren Richtung subchondral so eingebracht, dass in Bezug zu einer ventrodorsal ausgerichteten medialen Nullachse 78 eine Winkelabweichung epsilon von 30 Grad nach medial 77 oder 25 nach zentral 71 vorzugsweise von 0 Grad mit einer Abweichung von 5 Grad in jeder Richtung definiert und in Bezug zu einer ventrodorsal ausgerichteten lateralen Nullachse 79 eine Winkelabweichung zeta von 30 Grad nach lateral 72 und von 30 Grad nach zentral 71 vorzugsweise von 0 Grad mit einer Abweichung von je 5 Grad definiert sind (Fig. 5E).

In Fig. 6 sind die Fixationssektoren der endostalen Fixationselemente in der sagittalen Ebene dargestellt: Die endostalen lateralen (L), zentralen (Z) und medialen (M) Fixationselemente 25 (nicht gezeichnet) sind in der sagittalen Ebene im wesentlichen subchondral, parallel zur individuellen Dorsalneigung des medialen und lateralen Tibiaplateaus 101 ausgerichtet und bilden in Bezug zur sagittal ausgerichteten anteroposterioren Nullachse 102 eine Winkelabweichung aeta von 25 Grad nach cranial 103 und von 45 Grad nach caudal 104. Vorzugsweise ist der Winkel theta in einem Winkel theta von 0 bis 90 Grad plus minus 5 Grad zur nagelseitigen Klemmfläche 7 ("Fixationsmechanismus") des unter dem Ligamentum patellae lokalisierten, endomedullären Kraftträgers, d.h. des Marknagels 1 ausgelegt.

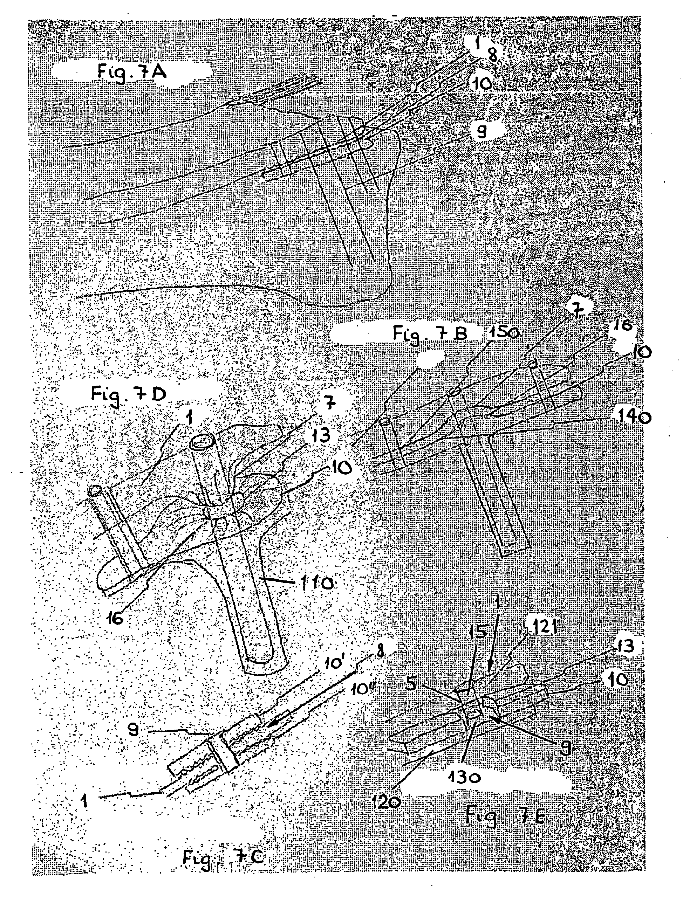

In den Fig. 7A,7B,7C,7D,7E sind die geeigneten Fixationsmechanismen zwischen der Basisplatte (BP) einerseits und dem endomedullärem Kraftträger (EMKT) andrerseits dargestellt:
Fein oberflächen-strukturierter Fixationsmechanismus Ausführungsvariante platten-förmig ausgelegt (Fig. 7A):
   In dieser Ausführungsvariante ist der reziproke Fixationsmechanismus vorzugsweise plattenförmig 8 ausgestaltet, so dass die in geeigneter Art feinstrukurierte Fixationsfläche der Knochenplatte 10 oder Basisplatte mit der ähnlich strukturierten Fixationsfläche des endomedullären Kraftträgers, bzw. Marknagels 1 zur gegenseitigen, winkelstabilen Fixation mittels eines Kompressionsmechanismus 9 beim Unterdruckbringen der Fixationsflächen eingreift.
Feinverzahnter Fixationsmechanismus Ausführungsvariante konvex-konkav ausgelegt (Fig. 7B):
   In dieser Ausführungsvariante ist der Fixationsmechanismus der Oberfläche der Knochenplatte 10, bzw. Basisplatte feinverzahnt und mit einer nach ventral vorzugsweise konvex gekrümmten plattenseitigen Klemmfläche 16 ausgeformt zur stabilen Fixation mit Feinverzahnungen 140 ausgelegt, die in die reziproke nach dorsal konkav ausgelegte, feinverzahnten nagelseitige Klemmfläche 7 auf der Unterfläche 150 des Marknagels 1, bzw. endomedullären Kraftträgers eingreifen.
Feinverzahnter Fixationsmechanismus Ausführungsvariante flach-flach ausgelegt (Fig. 7C):
   In dieser Fixationsvariante ist der Fixationsmechanismus der oberen Knochenplatte 10' und der unteren Knochenplatte 10" feinverzahnt ausgelegt und nach ventral vorzugsweise flach ausgeformt zur stabilen Fixation ausgelegt, die in den reziproken nach dorsal und ventral feinverzahnt flach ausgelegten Fixationsmechanismus 8 des Marknagels 1 bzw. endomedullären Kraftträgers eingreifen: Feinverzahnter,
   verschiebbarer Fixationsmechanismus, Unterdruckbringen der ineinandergreifenden, feinverzahnten Oberflächen durch zentral ausgelegte Kompressionsvorrichtung 9.
Fig. 7D zeigt einen Marknagel 1 und eine Knochenplatte 10 mit einem hülsenförmigen Fortsatz 110 zur Durchführung eines endostalen Knochenfixationselementes 25 durch die zentrale Öffnung 13. Ferner sind die nagelseitige und die plattenseitige Klemmfläche 7; 16 gekrümmt, wobei die nagelseitige Klemmfläche 7 konvex und die plattenseitige Klemmfläche 16 korrespondierend konkav gekrümmt ist.
Fig. 7E zeigt eine Ausführungsform, welche einen Marknagel 1 mit gabelförmig ausgebildetem proximalen Endteil 3 und eine zwischen den beiden Gabelzinken 120; 121 angeordnete Knochenplatte 10 umfasst. Die Kompressionsvorrichtung 9 zur Fixierung der Knochenplatte 10 am Marknagel 1 umfasst hier im wesentlichen eine Klemmschraube 15, welche durch die Bohrung 5 in der zweiten Gabelzinke 121 des Marknagels 1 und die zentrale Öffnung 13 in der Knochenplatte 10 durchgeführt wird und in einem korrespondierenden Innengewinde 130 in der ersten Gabelzinke 120 fixierbar ist.

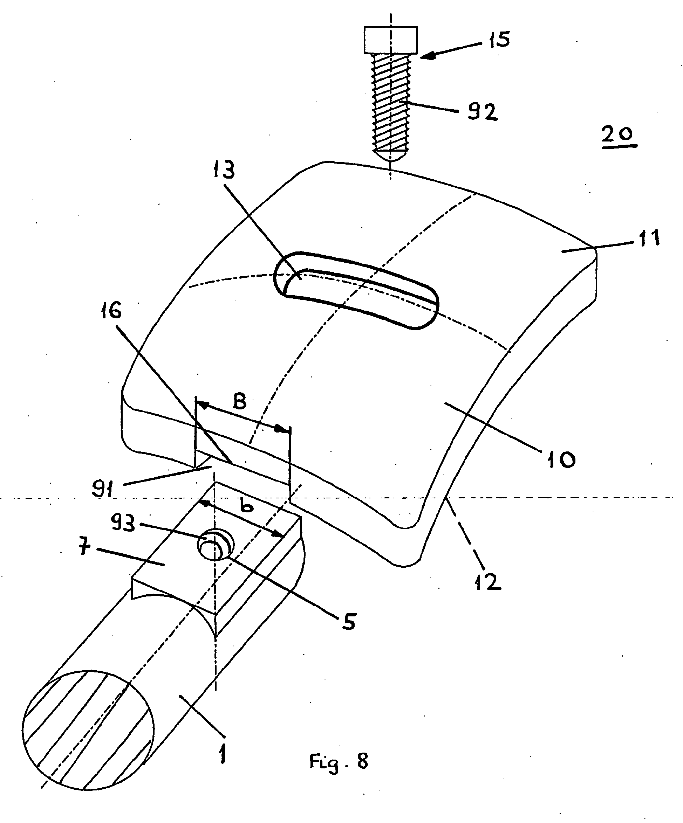

Fig. 8 zeigt eine Ausführungsform der Knochenfixationsvorrichtung 20 mit einer sphärisch gekrümmten Knochenplatte 10. Die Knochenplatte 10 umfasst an der Unterseite 12 eine Nute 91 mit einer Breite B, deren Nutengrund die plattenseitige Klemmfläche 16 bildet. Dazu ist die Nute 91 derart ausgebildet, dass der Nutengrund eine ebene Fläche ist. Der Nutengrund kann je nach Anforderung nur auf einem Teil der Nutenlänge eben ausgebildet sein. Die nagelseitige Klemmfläche 7 des Marknagels 1 hat eine Breite b < B, so dass die marknagelseitige Klemmfläche 7 relativ zur plattenseitigen Klemmfläche 16 verschiebbar ist. Ferner umfasst die Knochenplatte 10 eine von der Oberseite 11 zur Unterseite 12 durchgehende, zentrale Öffnung 13, welche zur Aufnahme des Schraubenschaftes 92 einer Klemmschraube 15 geeignet ist. Die Bohrung 5 im Marknagel 1 ist mit einem Innengewinde 93 versehen, so dass die Klemmschraube 15 ist die Bohrung 5 einschraubbar ist. Die Klemmschraube 15 bildet zusammen mit der durch das Innengewinde 93 gebildeten Gewindeverbindung die Kompressionsvorrichtung 9 für das Verklemmen der nagelseitigen und der plattenseitigen Klemmflächen 7,16.

Nachfolgend wird eine mögliche Operationstechnik für die erfindungsgemässe Knochenfixationsvorrichtung beschrieben, um deren Funktion besser hervortreten zu lassen. Die Operationstechnik wird am Beispiel einer Tibia beschrieben.

### Operationstechnik

1. Inzision lateral des Ligamentum Patellae, was den Vorteil aufweist, dass der Streckapparat nicht zerstört wird.
2. Aufsetzen einer Kernbohrung in der vorwiegend antero-posterioren Richtung, parallel zur individuellen Neigung des Tibiaplateaus in der Sagittalebene. Die Bohrungsachse 6 entspricht gleichzeitig der späteren Rotationsachse zwischen Marknagel 1 und Knochenplatte 10. Die Lokalisation erfolgt ausserhalb der Weichteilansätze im zentralen bis medialen Teil des lateralen Schienbeinkopfes in der Nähe der ehemaligen Wachstumsfugenlinie (proximale Tibiaepiphysenlinie) bis zur hinteren Kortikalis.
3. Entnahme und Aufbewahrung des derart gebildeten Knochenzylinders.
4. Anlegen einer inkompletten, horizontalen Knochenschwächung ausgehend von der Kernbohrung und nach lateral, oberhalb des proximalen Tibiofibular-Gelenkes lokalisiert mit genügendem Abstand zur subchondralen Knochenplatte des lateralen Schienbeinkopfes.
5. Durchführung einer unvollständigen Unterschneidung der Tuberositas tibiae, die im wesentlichen mit der lateralen Säule der Tibia verbunden bleibt.
6. Anlegen einer von der ersten Kernbohrung ausgehenden zweiten Kernbohrung, welche im wesentlichen in Richtung Markhöhle gerichtet ist.
7. Entnahme und Aufbewahrung des so gewonnenen Knochenzylinders.
8. Eine Zentrierbohrhülse wird in die zweite Kernbohrung eingebracht, so dass die schräge Ebene der gezielten Knochenschwächung und die Neigung derselben in Bezug zur Längsachse der Tibia - vorzugsweise unter Bildwandlerkontrolle - festgelegt werden kann.
9. Inzision im Bereich der postero-medialen Schienbeinrundung.
10. Abscheiben des Periostes auf kurzer Strecke.
11. Führen der Schneidwerkzeuge entsprechend den angelegten Kernbohrungen.
12. Kreieren einer Knochenöffnung oder eines inkompletten Knochenspaltes im Bereich der durch die Knochenschwächungen vorgegebenen Ebenen.
13. Kontrolle der mechanischen Beinachse, definiert als Verbindungslinie zwischen Hüftkopfzentrum und oberer Sprunggelenksmitte, derart dass der das Kniegelenk überkreuzende Kraftvektor vom beschädigten Kniegelenkskompartiment in das nicht oder weniger beschädigte Kniegelenkskompartiment durch progredientes Aufspreizen der Knochenteile verschoben wird.
14. Nach erfolgter Verifikation des Verlaufs der neuen mechanischen Beinachse Fixation der Relativposition der aufgespreizten Knochenteile gegeneinander bis zur sicheren knöchernen Konsolidierung mittels der erfindungsgemässen Knochenfixationsvorrichtung.

Bezüglich der Verfahrensschritte 2 und 8 erfolgen diese vorzugsweise unter Kontrolle der Lage und Orientierung der ersten Kernbohrung mittels Bildwandler-Fluoronavigation, landmarkenspezifischen, computerassistierten Technologien.

Weitere Varianten der Knochenschwächung bestehen in einer Z-förmigen Knochenschwächung für eine rechtsseitige Tibia und in einer umgekehrten Z-förmigen Knochenschwächung für eine linksseitige Tibia.

## Patentansprüche

1. Knochenfixationsvorrichtung mit
A) einem Marknagel (1) mit einem distalen Endteil (2), einem proximalen Endteil (3), einer Zentrallinie (4) und einer im proximalen Endteil (3) quer zur Zentrallinie (4) verlaufenden Bohrung (5) mit der Bohrungsachse (6); sowie
B) einer Knochenplatte (10) mit einer Oberseite (11), einer für den Knochenkontakt bestimmten Unterseite (12), einer im zentralen Teil der Knochenplatte (10) untergebrachten, die Oberseite (11) mit der Unterseite (12) verbindenden, durchgehenden, zentralen Öffnung (13) und mehreren, peripher um die zentrale Öffnung (13) angeordneten, ebenfalls durchgehenden Plattenlöcher (14),
**dadurch gekennzeichnet, dass**
C) am proximalen Endteil (3) des Marknagels (1) mindestens eine nagelseitige Klemmfläche (7) ausgebildet ist; und
D) die Knochenplatte (10) mindestens eine zur nagelseitigen Klemmfläche (7) korrespondierende plattenseitige Klemmfläche (16) aufweist; so dass
E) die beiden zueinander korrespondierenden Klemmflächen (7,16) im ungeklemmten Zustand gegeneinander verschiebbar sind und im geklemmten Zustand gegeneinander fixiert sind.

2. Knochenfixationsvorrichtung nach Anspruch 1, **dadurch gekennzeichnet, dass** die Knochenfixationsvorrichtung eine Kompressionsvorrichtung (9) umfasst, mittels welcher die nagelseitige und die plattenseitige Klemmfläche (7,16) gegeneinander pressbar sind.

3. Knochenfixationsvorrichtung nach Anspruch 2, **dadurch gekennzeichnet, dass** die Kompressionsvorrichtung (9) ein Innengewinde (93) in der Bohrung (5) und eine durch die zentrale Öffnung (13) hindurchführbare und in das Innengewinde (93) der Bohrung (5) schraubbare Klemmschraube (15) umfasst.

4. Knochenfixationsvorrichtung nach einem der Ansprüche 1 bis 3, **dadurch gekennzeichnet, dass** der Marknagel (1) zwei nagelseitige Klemmflächen (7) aufweist.

5. Knochenfixationsvorrichtung nach Anspruch 4, **dadurch gekennzeichnet, dass** die zwei nagelseitgen Klemmflächen (7) im wesentlichen asymmetrisch exzentrisch zur Zentrallinie (4) angeordnet sind.

6. Knochenfixationsvorrichtung nach einem der Ansprüche 1 bis 5, **dadurch gekennzeichnet, dass** die nagelseitige Klemmfläche (7) eben ausgebildet ist und eine Ebene B2 definiert, weiche mit einer durch die Zentrallinie (4) und den parallel zur Ebene B2 verlaufenden Durchmesser d des Marknagels (1) definierten Ebene B1 einen Winkel alpha von 0° bis 30°, vorzugsweise von 10° bis 20° aufweist.

7. Knochenfixationsvorrichtung nach einem der Ansprüche 1 bis 5, **dadurch gekennzeichnet, dass** eine zur Bohrungsachse (6) orthogonale Ebene B2 mit einer durch die Zentrallinie (4) und den parallel zur Ebene B2 verlaufenden Durchmesser d des Marknagels (1) definierten Ebene B1 einen Winkel alpha von 0° bis 30°, vorzugsweise von 10° bis 20° aufweist.

8. Knochenfixationsvorrichtung nach einem der Ansprüche 1 bis 5, **dadurch gekennzeichnet, dass** eine zur Bohrungsachse (6) orthogonale Ebene B2 parallel zu einer durch die Zentrallinie (4) und den parallel zur Ebene B2 verlaufenden Durchmesser d des Marknagels (1) definierten Ebene B1 verläuft.

9. Knochenfixationsvorrichtung nach einem der Ansprüche 1 bis 8, **dadurch gekennzeichnet, dass** die nagelseitige Klemmfläche (7) im Bereich der Bohrung (5) gelegen ist.

10. Knochenfixationsvorrichtung nach einem der Ansprüche 1 bis 9, **dadurch gekennzeichnet, dass** die plattenseitige Klemmfläche (16) an der Unterseite (12) der Knochenplatte (10) ausgebildet ist.

11. Knochenfixationsvorrichtung nach einem der Ansprüche 1 bis 9, **dadurch gekennzeichnet, dass** die plattenseitige Klemmfläche (16) an der Oberseite (11) der Knochenplatte (10) ausgebildet ist.

12. Knochenfixationsvorrichtung nach Anspruch 10 und 11, **dadurch gekennzeichnet, dass** sowohl die Oberseite (11) als auch die Unterseite (12) der Knochenplatte (10) eine plattenseitige Klemmfläche (16) aufweisen.

13. Knochenfixationsvorrichtung nach einem der Ansprüche 1 bis 12, **dadurch gekennzeichnet, dass** die plattenseitige Klemmfläche (16) im Inneren der Knochenplatte (10) ausgebildet ist und im wesentlichen parallel zu ihrer Unter- und Oberseite (11,12) verläuft.

14. Knochenfixationsvorrichtung nach einem der Ansprüche 1 bis 13, **dadurch gekennzeichnet, dass** die plattenseitige Klemmfläche (16) im Bereich der Öffnung (13) ausgebildet ist.

15. Knochenfixationsvorrichtung nach einem der Ansprüche 1 bis 14, **dadurch gekennzeichnet, dass** die Zentrallinie (4) die nagelseitige Klemmfläche (7) unter einem durchschnittlichen Winkel von 1° - 20°, vorzugsweise von 2° -10° schneidet.

16. Knochenfixationsvorrichtung nach einem der Ansprüche 1 bis 15, **dadurch gekennzeichnet, dass** der proximale Endteil (3) des Marknagels (1) als separates, modulares Bauteil ausgebildet.

17. Knochenfixationsvorrichtung nach einem der Ansprüche 1 bis 16, **dadurch gekennzeichnet, dass** zwischen den zueinander korrespondierenden Klemmflächen (7,16) ein Zwischenstück (30) aus einem plastisch deformierbaren Material angeordnet ist.

18. Knochenfixationsvorrichtung nach einem der Ansprüche 1 bis 17, **dadurch gekennzeichnet, dass** der Marknagel (1) asymmetrisch, multiplanar gekrümmt ist.

19. Knochenfixationsvorrichtung nach einem der Ansprüche 1 bis 18, **dadurch gekennzeichnet, dass** die zueinander korrespondierenden Klemmflächen (7,16) je planar oder sphärisch oder zylindrisch ausgebildet sind.

20. Knochenfixationsvorrichtung nach einem der Ansprüche 1 bis 19, **dadurch gekennzeichnet, dass** die Klemmflächen (7,16) eine dreidimensionale Strukturierung aufweisen, vorzugsweise in Form von Verzahnungen

21. Knochenfixationsvorrichtung nach Anspruch 20, **dadurch gekennzeichnet, dass** die Verzahnungen in Form von abgerundeten Windungen, Pyramidenspitzen oder von vielschichtigen Polygonen ausgebildet sind.

22. Knochenfixationsvorrichtung nach einem der Ansprüche 1 bis 21, **dadurch gekennzeichnet, dass** das distale Marknagelende parabol symmetrisch abgerundet ist.

23. Knochenfixationsvorrichtung nach einem der Ansprüche 1 bis 22, **dadurch gekennzeichnet, dass** der Marknagel (1) mindestens neun planparallel angeordnete Längsrillen aufweist.

24. Knochenflxationsvorrichtung nach einem der Ansprüche 1 bis 23, **dadurch gekennzeichnet, dass** das distale Ende des Marknagels (1) längs gespalten ist.

25. Knochenfixationsvorrichtung nach einem der Ansprüche 1 bis 24, **dadurch gekennzeichnet, dass** die Knochenplatte (10) eine konkave, gekrümmte Unterseite (12) aufweist.

26. Knochenfixationsvorrichtung nach einem der Ansprüche 1 bis 25, **dadurch gekennzeichnet, dass** die Knochenplatte (10) eine konvexe, gekrümmt Oberseite (11) aufweist.

27. Knochenfixationsvorrichtung nach Anspruch 25 oder 26, **dadurch gekennzeichnet, dass** die Unterseite (12) und/oder die Oberseite (11) sphärisch gekrümmt ausgebildet sind.

28. Knochenfixationsvorrichtung nach einem der Ansprüche 1 bis 27, **dadurch gekennzeichnet, dass** die Unterseite (12) der Knochenplatte (10) eine Nute (91) mit einem die plattenseitige Klemmfläche (16) bildenden Nutenboden, einer ersten und einer zweiten Seitenwand aufweist, und dass Nute (91) zwischen der ersten und der zweiten Seitenwand eine minimale Breite B aufweist, welche eine mindestens teilweise Aufnahme des proximalen Endteils (3) des Marknagels (1) im Bereich der nagelseitigen Klemmfläche (7) gestattet, so dass die nagelseitige Klemmfläche (7) an der plattenseitigen Klemmfläche (16) zur Anlage kommt.

29. Knochenfixationsvorrichtung nach Anspruch 28, **dadurch gekennzeichnet, dass** das proximale Nagelende (3) des Marknagels (1) im Bereich der nagelseitigen Klemmfläche (7) eine Breite b < B aufweist.

30. Knochenfixationsvorrichtung nach einem der Ansprüche 1 bis 29, **dadurch gekennzeichnet, dass** die Knochenfixationsvorrichtung mindestens ein eine Längsachse aufweisendes Knochenfixationselement (25) umfasst.

31. Knochenfixationsvorrichtung nach einem der Ansprüche 6, 7 oder 8 und 30, **dadurch gekennzeichnet, dass** das mindestens eine Knochenfixatonselement (25) derart angeordnet ist, dass die Projektionen seiner Längsachse in die X,Z-Ebene, die X,Y-Ebene und in die Y,Z-Ebene eines dreidimensionalen Koordinatensystems mit den Achsen x,y,z relativ zur x-Achse (in der X,Y-Ebene), zur z-Achse (in der X,Z-Ebene) und zur z-Achse (in der Y,Z-Ebene) einen Winkel zwischen 0° und 60° einschliessen, wobei die z-Achse koaxial zur Zentrallinie (4) des Marknagels (1) im Bereich der nagelseitigen Klemmfläche (7) verläuft und die x-Achse orthogonal zur z-Achse und koaxial zu dem parallel zur Ebene (2) verlaufenden Durchmesser d des Marknagels (1) verläuft.

32. Knochenfixationsvorrichtung nach einem der Ansprüche 6, 7 oder 8 und 30, **dadurch gekennzeichnet, dass** die z-Achse - bei einem Marknagel (1) mit gekrümmter Zentrallinie (4) - durch die Tangente an die Zentrallinie (4) im Schnittpunkt der Zentrallinie (4) mit der die Drehachse bildenden Bohrungsachse (6) gebildet wird.

33. Knochenfixationsvorrichtung nach einem der Ansprüche 1 bis 32, **dadurch gekennzeichnet, dass** die Oberfläche ihrer Bauteile, insbesondere des Marknagels (1), der Knochenplatte (10) sowie allfälliger Knochenfixationselemente (25), funkenanodisiert sind, vorzugsweise mittels anodischer Funkenanodisierung II.

34. Knochenfixationsvorrichtung nach einem der Ansprüche 1 bis 33, **dadurch gekennzeichnet, dass** die Oberfläche ihrer Bauteile, insbesondere des Marknagels (1), der Knochenplatte (10) sowie allfälliger Knochenfixationselemente (25) mit einer - vorzugsweise Silberionen enthaltenden - mikrobiziden Beschichtung versehen ist.

35. Knochenfixationsvorrichtung nach einem der Ansprüche 1 bis 34, **dadurch gekennzeichnet, dass** der Marknagel (1) asymmetrisch ausgebildet ist.

36. Knochenfixationsvorrichtung nach einem der Ansprüche 1 bis 35, **dadurch gekennzeichnet, dass** der Marknagel (1) in einer ersten (frontalen oder latero-medialen) Ebene eine erste Krümmung und in einer, senkrecht zur ersten Ebene stehenden, zweiten (sagittalen oder antero-posterioren) Ebene eine zweite Krümmung aufweist.

37. Knochenfixationsvorrichtung nach einem der Ansprüche 1 bis 36, **dadurch gekennzeichnet, dass** sie zusätzlich eines oder mehrere der folgenden Bauteile:
- eine Stoss-Schraube
- eine Zug-Schraube
- eine Kompressions-Schraube
- eine Stellschraube
als Knochenfixationselemente (25) umfasst

38. Knochenfixationsvorrichtung nach einem der Ansprüche 1 bis 37, **dadurch gekennzeichnet, dass** der Marknagel (1) eine vorzugsweise durchgehende Kannulierung aufweist.

39. Knochenfixationsvorrichtung nach einem der Ansprüche 1 bis 38, **dadurch gekennzeichnet, dass** die Knochenfixationselemente (25) als elastische Markraumdrähte ausgebildet sind.

40. Knochenfixationsvorrichtung nach einem der Ansprüche 1 bis 38, **dadurch gekennzeichnet, dass** die Knochenfixationselemente (25) Hülsen (26) umfassen, welche in Aufnahmen (28) in der Knochenplatte (10) eingepresst sind und deren Zentralbohrungen die Durchführung von Knochenschrauben gestatten.

## Claims

1. Bone fixation device with
A) a medullary nail (1) with a distal extremity (2), a proximal extremity (3), a central line (4) and a borehole (5) with the borehole axis (6) extending across the central line (4) in the proximal extremity (3), and
B) a bone plate with an upper side (11), a lower side (12) destined to contact the bone, a continuous central opening (13) set in the central portion of the bone plate (10) and connecting the upper side (11) with the lower side (12), and several similarly continuous plate holes (14) set up peripherally around the central opening (13),
**characterized by the fact that**
C) at least one clamping surface (7) is conformed at the side of the nail on the proximal extremity (3) of the medullary nail (1); and
D) the bone plate (10) presents at least one clamping surface (16) corresponding to the clamping surface (7) at the side of the nail; so that
E) the two clamping surfaces corresponding to each other (7,16) are displaceable against each other in an unclamped condition, and fixed to each other in a clamped condition.

2. Bone fixation device according to claim 1, **characterized by** the fact that the bone fixation device comprises a compression device (9), by which the clamping surfaces (7, 16) on the side of the nail and on the side of the plate can be pressed against each other.

3. Bone fixation device according to claim 2, **characterized by** the fact that the compression device (9) comprises an internal thread (93) in the borehole (5), and a clamping screw (15) that can be passed through the central opening (13) and screwed into the internal thread (93) of the borehole (5).

4. Bone fixation device according to one of the claims from 1 to 3, **characterized by** the fact that the medullary nail (1) presents two clamping surfaces (7) at the side of the nail.

5. Bone fixation device according to claim 4, **characterized by** the fact that the two clamping surfaces (7) on the side of the nail are essentially disposed in a way asymmetrically eccentric to the central line (4).

6. Bone fixation device according to one of the claims from 1 to 5, **characterized by** the fact that the clamping surface (7) at the side of the nail is conformed in a flat manner and defines a plane B2 that forms an angle alpha from 0° to 30°, preferably from 10° to 30°, with the plane B1 which is defined by the central line (4) and the medullary nail's (1) diameter d extending parallel to the plane B2.

7. Bone fixation device according to one of the claims from 1 to 5, **characterized by** the fact that a surface B2 orthogonal to the borehole axis (6) presents an angle alpha of 0° to 30°, preferably of 10° to 20° with respect to a plane B1 defined by the central line (4) and the medullary nail's (1) diameter d running parallel to the plane B2.

8. Bone fixation device according to one of the claims from 1 to 5, **characterized by** the fact that a plane B2 orthogonal to the borehole axis (6) extends parallel to a plane B1 that is defined by the central line (4) and the medullary nail's (1) diameter d running parallel to the plane B2.

9. Bone fixation device according to one of the claims from 1 to 8, **characterized by** the fact that the clamping surface (7) at the side of the nail is set within the area of the borehole (5).

10. Bone fixation device according to one of the claims from 1 to 9, **characterized by** the fact that the clamping surface (16) at the side of the plate is conformed on the lower side (12) of the bone plate (10).

11. Bone fixation device according to one of the claims from 1 to 9, **characterized by** the fact that clamping surface (16) at the side of the plate is conformed on the upper side (11) of the bone plate (10).

12. Bone fixation device according to the claims 10 and 11, **characterized by** the fact that both the upper side (11) as well as the lower side (12) of the bone plate (10) present a clamping surface (16) at the side of the plate.

13. Bone fixation device according to one of the claims from 1 to 12, **characterized by** the fact that the clamping surface (16) at the side of the plate is conformed in the interior of the bone plate (10) and runs in a direction essentially parallel direction to its lower side and upper side (11, 12).

14. Bone fixation device according to one of the claims from 1 to 13, **characterized by** the fact that the clamping surface (16) at the side of the plate is conformed in the area of the opening (13).

15. Bone fixation device according to one of the claims from 1 to 14, **characterized by** the fact that the central line (4) crosses the clamping surface (7) at the side of the nail at an average angle from 1° to 20°, preferably from 2° to 10°.

16. Bone fixation device according to one of the claims from 1 to 15, **characterized by** the fact that the proximal extremity (3) of the medullary nail (1) is conformed as a separate, modular construction element.

17. Bone fixation device according to one of the claims from 1 to 16, **characterized by** the fact that an intermediate element (30) made of a plastically deformable material is set up between the clamping surfaces (7, 16) corresponding to each other.

18. Bone fixation device according to one of the claims from 1 to 17, **characterized by** the fact that the medullary nail (1) is bent in an asymmetrical, multi-planar manner.

19. Bone fixation device according to one of the claims from 1 to 18, **characterized by** the fact that the clamping surfaces (7, 16) corresponding to each other are each conformed in a planar, spherical, or cylindrical manner.

20. Bone fixation device according to one of the claims from 1 to 19, **characterized by** the fact that the clamping surfaces (7, 16) present a tridimensional structure, preferably in the form of indentations.

21. Bone fixation device according to claim 20, **characterized by** the fact that the indentations are conformed in the way of rounded windings, pyramid tips or multilayered polygons.

22. Bone fixation device according to one of the claims from 1 to 21, **characterized by** the fact that the medullary nail's distal extremity is symmetrically rounded.

23. Bone fixation device according to one of the claims from 1 to 22, **characterized by** the fact that the medullary nail (1) presents at least nine longitudinal grooves set up parallel to the plane.

24. Bone fixation device according to one of the claims from 1 to 23, **characterized by** the fact that the medullary nail's (1) distal extremity is longitudinally split.

25. Bone fixation device according to one of the claims from 1 to 24, **characterized by** the fact that the bone plate (10) presents a bent, concave lower side (12).

26. Bone fixation device according to one of the claims from 1 to 25, **characterized by** the fact that the bone plate (10) presents a bent, convex upper side (11).

27. Bone fixation device according to the claim 25 or 26, **characterized by** the fact that the lower side (12) and/or the upper side (11) are conformed in a spherically bent manner.

28. Bone fixation device according to one of the claims from 1 to 27, **characterized by** the fact that the bone plate's (10) lower side (12) presents a groove (91) with a groove bottom forming the clamping surface (16) at the side of the plate and a first and a second lateral wall, and that the groove (91) between the first and the second lateral wall presents a minimum width B that allows at least a partial reception of the medullary nail's (1) proximal extremity (3) in the area of the clamping surface (7) at the side of the nail, so that the clamping surface (7) at the side of the nail comes to rest on the clamping surface (18) at the side of the plate.

29. Bone fixation device according to claim 28, **characterized by** the fact that the medullary nail's (1) proximal extremity presents a width B < b in the area of the clamping surface (7) at the side of the nail.

30. Bone fixation device according to one of the claims from 1 to 29, **characterized by** the fact that then bone fixation device comprises at least one bone fixation element (25) presenting a longitudinal axis.

31. Bone fixation device according to one of the claims 6, 7 or 8 and 30, **characterized by** the fact that the at least one bone fixation element (25) is set up so that the projections of its longitudinal axis into the X, Z plane, the X,Y plane and the Y, Z plane of a three-dimensional system of coordinates enclose an angle between 0° and 60° with the axes x,y,z relative to the x-axis (in the X,Y plane), to the y-axis (in the X, Z plane) and to the z-axis (in the Y, Z plane), where the z-axis extends coaxially to the medullary nail's (1) central line (4) in the area of the clamping surface (7) at the side of the nail, and the x-axis extends orthogonally to the z-axis and coaxially to the medullary nail's (1) diameter d running parallel to the plane (2).

32. Bone fixation device according to one of the claims 6, 7 or 8 and 30, **characterized by** the fact that the z-axis - in a medullary nail (1) with a bent central line - is formed by the tangent to the central line (4) in the crossing point of the central line (4) with the borehole axis (6) that forms the rotational axis.

33. Bone fixation device according to one of the claims from 1 to 32, **characterized by** the fact that the upper surface of its construction elements, in particular of the medullary nail (1), the bone plate (10) and eventual other bone fixation elements (25) is spark-anodized, preferably by using a spark anodizing system II.

34. Bone fixation device according to one of the claims from 1 to 33, **characterized by** the fact that the upper surface of its construction elements, in particular of the medullary nail (1), the bone plate (10) and eventual other bone fixation elements (25) is provided with a microbiocidal coating preferably containing silver ions.

35. Bone fixation device according to one of the claims from 1 to 34, **characterized by** the fact that the medullary nail (1) is conformed in an asymmetrical manner.

36. Bone fixation device according to one of the claims from 1 to 35, **characterized by** the fact that the medullary nail (1) presents a first bend in a first (frontal or lateral-median) plane, and a second bend in a second (sagittal or anterior-posterior) plane set up vertically to the first plane.

37. Bone fixation device according to one of the claims from 1 to 38, **characterized by** the fact that it additionally comprises one or several of the following construction elements as bone fixation elements (25):
- a thrust screw
- a traction screw
- a compression screw
- a set screw

38. Bone fixation device according to one of the claims from 1 to 37, **characterized by** the fact that the medullary nail (1) presents an essentially continuous groove.

39. Bone fixation device according to one of the claims from 1 to 38, **characterized by** the fact that the bone fixation elements (25) are conformed as elastic medullary space wires.

40. Bone fixation device according to one of the claims from 1 to 38, **characterized by** the fact that the bone fixation elements (25) comprise bushings (26) that are pressure-set into the receptacles (28) in the bone plate (10), and whose central boreholes allow passing bone screws across them.

## Revendications

1. Dispositif de fixation osseuse comprenant
A) un clou intramédullaire (1) présentant une extrémité distale (2), une extrémité proximale (3), une ligne centrale (4) et un alésage (5) avec son axe d'alésage (6), lequel alésage s'étend transversalement à la ligne centrale (4) dans l'extrémité proximale (3) ; et
B) une plaque d'ostéosynthèse (10) présentant une face supérieure (11), une face inférieure (12) destinée au contact avec l'os, une ouverture centrale traversante (13) pratiquée dans la partie centrale de la plaque d'ostéosynthèse (10), reliant la face supérieure (11) avec la face inférieure (12) et plusieurs ouvertures de plaque (14), également traversantes, situées à la périphérie de l'ouverture centrale (13),
**caractérisé en ce que,**
C) au moins une surface de serrage (7) appartenant au clou est réalisée au niveau de l'extrémité proximale (3) du clou intramédullaire (1) ; et
D) la plaque d'ostéosynthèse (10) présente au moins une surface de serrage (16) appartenant à la plaque, laquelle surface correspond à la surface de serrage (7) du clou ; de sorte que
E) à l'état non serré, les deux surfaces de serrage (7,16) correspondant l'une à l'autre peuvent être déplacées l'une par rapport à l'autre et, à l'état serré, elles sont bloquées l'une par rapport à l'autre.

2. Dispositif de fixation osseuse selon la revendication 1, **caractérisé en ce que** le dispositif de fixation osseuse comprend un dispositif de compression (9), au moyen duquel la surface de serrage appartenant au clou et la surface de serrage appartenant à la plaque (7,16) peuvent être pressées l'une contre l'autre.

3. Dispositif de fixation osseuse selon la revendication 2, **caractérisé en ce que** le dispositif de compression (9) comprend un taraudage (93) dans l'alésage (5) et une vis de serrage (15) pouvant être introduite dans l'ouverture centrale (13) et vissée dans le taraudage (93) de l'alésage (5).

4. Dispositif de fixation osseuse selon l'une quelconque des revendications 1 à 3, **caractérisé en ce que** le clou intramédullaire (1) présente deux surfaces de serrage (7) appartenant au clou.

5. Dispositif de fixation osseuse selon la revendication 4, **caractérisé en ce que** les deux surfaces de serrage (7) appartenant au clou sont disposées essentiellement de manière excentrique et asymétrique par rapport à la ligne centrale (4).

6. Dispositif de fixation osseuse selon l'une quelconque des revendications 1 à 5, **caractérisé en ce que** la surface de serrage (7) appartenant au clou est réalisée de manière plane et définit un plan B2, lequel plan présente, par rapport à un plan B1 défini par la ligne centrale (4) et le diamètre d du clou intramédullaire (1) s'étendant parallèlement au plan B2, un angle alpha allant de 0° à 30°, de préférence de 10° à 20°.

7. Dispositif de fixation osseuse selon l'une quelconque des revendications 1 à 5, **caractérisé en ce qu'**un plan B2 orthogonal à l'axe d'alésage (6) présente, par rapport à un plan B1 défini par la ligne centrale (4) et le diamètre d du clou intramédullaire (1) s'étendant parallèlement au plan B2, un angle alpha allant de 0° à 30°, de préférence de 10° à 20°.

8. Dispositif de fixation osseuse selon l'une quelconque des revendications 1 à 5, **caractérisé en ce qu'**un plan B2 orthogonal à l'axe d'alésage (6) s'étend parallèlement à un plan B1 défini par la ligne centrale (4) et le diamètre d du clou intramédullaire (1) s'étendant parallèlement au plan B2.

9. Dispositif de fixation osseuse selon l'une quelconque des revendications 1 à 8, **caractérisé en ce que** la surface de serrage appartenant au clou (7) est située dans la zone de l'alésage (5).

10. Dispositif de fixation osseuse selon l'une quelconque des revendications 1 à 9, **caractérisé en ce que** la surface de serrage appartenant à la plaque (16) est réalisée au niveau de la face inférieure (12) de la plaque d'ostéosynthèse (10).

11. Dispositif de fixation osseuse selon l'une quelconque des revendications 1 à 9, **caractérisé en ce que** la surface de serrage appartenant à la plaque (16) est réalisée au niveau de la face supérieure (11) de la plaque d'ostéosynthèse (10).

12. Dispositif de fixation osseuse selon la revendication 10 et 11, **caractérisé en ce que** la face supérieure (11) ainsi que la face inférieure (12) de la plaque d'ostéosynthèse (10) présentent chacune une surface de serrage (16) appartenant à la plaque.

13. Dispositif de fixation osseuse selon l'une quelconque des revendications 1 à 12, **caractérisé en ce que** la surface de serrage (16) appartenant à la plaque est réalisée à l'intérieur de la plaque d'ostéosynthèse (10) et s'étend essentiellement parallèlement à la face inférieure et supérieure (11,12) de celle-ci.

14. Dispositif de fixation osseuse selon l'une quelconque des revendications 1 à 13, **caractérisé en ce que** la surface de serrage (16) appartenant à la plaque est réalisée dans la zone de l'ouverture (13).

15. Dispositif de fixation osseuse selon l'une quelconque des revendications 1 à 14, **caractérisé en ce que** la ligne centrale (4) coupe la surface de serrage (7) appartenant au clou selon un angle moyen allant de 1° à 20°, de préférence de 2° à 10°.

16. Dispositif de fixation osseuse selon l'une quelconque des revendications 1 à 15, **caractérisé en ce que** l'extrémité proximale (3) du clou intramédullaire (1) est réalisée en tant qu'élément modulaire séparé.

17. Dispositif de fixation osseuse selon l'une quelconque des revendications 1 à 16, **caractérisé en ce qu'**un élément intermédiaire (30) fait d'une matière déformable de manière plastique est disposé entre les surfaces de serrage (7,16) correspondantes.

18. Dispositif de fixation osseuse selon l'une quelconque des revendications 1 à 17, **caractérisé en ce que** le clou intramédullaire (1) est cintré de manière multiplanaire asymétrique.

19. Dispositif de fixation osseuse selon l'une quelconque des revendications 1 à 18, **caractérisé en ce que** les surfaces de serrage correspondantes (7,16) sont réalisées chacune de forme planaire ou sphérique ou cylindrique.

20. Dispositif de fixation osseuse selon l'une quelconque des revendications 1 à 19, **caractérisé en ce que** les surfaces de serrage (7,16) présentent une texture tridimensionnelle, de préférence sous la forme de dentures.

21. Dispositif de fixation osseuse selon la revendication 20, **caractérisé en ce que** les dentures sont réalisées sous la forme de sinuosités arrondies, de pointes de diamant ou de polygones complexes.

22. Dispositif de fixation osseuse selon l'une quelconque des revendications 1 à 21, **caractérisé en ce que** l'extrémité distale du clou intramédullaire est arrondie en forme de paraboloïde de révolution.

23. Dispositif de fixation osseuse selon l'une quelconque des revendications 1 à 22, **caractérisé en ce que** le clou intramédullaire (1) présente au moins neuf rainures longitudinales disposées de manière plane et parallèle.

24. Dispositif de fixation osseuse selon l'une quelconque des revendications 1 à 23, **caractérisé en ce que** l'extrémité distale du clou intramédullaire (1) est fendue dans le sens de la longueur.

25. Dispositif de fixation osseuse selon l'une quelconque des revendications 1 à 24, **caractérisé en ce que** la plaque d'ostéosynthèse (10) présente une face inférieure (12) courbe concave.

26. Dispositif de fixation osseuse selon l'une quelconque des revendications 1 à 25, **caractérisé en ce que** la plaque d'ostéosynthèse (10) présente une face supérieure (11) courbe convexe.

27. Dispositif de fixation osseuse selon la revendication 25 ou 26, **caractérisé en ce que** la face inférieure (12) et/ou la face supérieure (11) sont réalisées avec une courbure sphérique.

28. Dispositif de fixation osseuse selon l'une quelconque des revendications 1 à 27, **caractérisé en ce que** la face inférieure (12) de la plaque d'ostéosynthèse (10) présente une rainure (91) comprenant un fond de rainure formant la surface de serrage appartenant à la plaque (16), une première et une deuxième paroi latérale, et **en ce que** la rainure (91) présente, entre la première et la deuxième paroi latérale, une largeur minimale B, laquelle permet un logement au moins partiel de l'extrémité proximale (3) du clou intramédullaire (1) dans la zone de la surface de serrage (7) appartenant au clou, de sorte que la surface de serrage appartenant au clou (7) vient en contact avec la surface de serrage (16) appartenant à la plaque.

29. Dispositif de fixation osseuse selon la revendication 28, **caractérisé en ce que**, dans la zone de la surface de serrage (7) appartenant au clou, l'extrémité proximale (3) du clou intramédullaire (1) présente une largeur b inférieure à B.

30. Dispositif de fixation osseuse selon l'une quelconque des revendications 1 à 29, **caractérisé en ce que** le dispositif de fixation osseuse comprend au moins un élément de fixation osseuse (25) présentant un axe longitudinal.

31. Dispositif de fixation osseuse selon l'une quelconque des revendications 6, 7 ou 8 et 30, **caractérisé en ce que** le au moins un élément de fixation osseuse (25) est disposé de telle sorte que les projections de son axe longitudinal sur le plan X,Z, sur le plan X,Y et sur le plan Y,Z d'un système de coordonnées tridimensionnel ayant pour axes x, y, z forment des angles avec l'axe x (dans le plan X,Y), avec l'axe z (dans le plan X,Z) et avec l'axe z (dans le plan Y,Z), qui sont compris entre 0° et 60°, dans lequel l'axe z s'étend coaxialement à la ligne centrale (4) du clou intramédullaire (1) dans la zone de la surface de serrage (7) appartenant au clou et l'axe x s'étend orthogonalement à l'axe z et coaxialement au diamètre d du clou intramédullaire (1) s'étendant parallèlement au plan (2).

32. Dispositif de fixation osseuse selon l'une quelconque des revendications 6, 7 ou 8 et 30, **caractérisé en ce que**, pour un clou intramédullaire (1) ayant une ligne centrale courbe (4), l'axe z est formé par la tangente à la ligne centrale (4) au niveau du point d'intersection de la ligne centrale (4) avec l'axe d'alésage (6) formant l'axe de rotation.

33. Dispositif de fixation osseuse selon l'une quelconque des revendications 1 à 32, **caractérisé en ce que** les surfaces de ses éléments, en particulier celle du clou intramédullaire (1), de la plaque d'ostéosynthèse (10) ainsi que d'éventuels éléments de fixation osseuse (25), sont traitées par oxydation anodique par décharge d'étincelle (ANOF), de préférence au moyen d'une oxydation anodique par décharge d'étincelle II.

34. Dispositif de fixation osseuse selon l'une quelconque des revendications 1 à 33, **caractérisé en ce que** les surfaces de ses éléments, en particulier celle du clou intramédullaire (1), de la plaque d'ostéosynthèse (10) ainsi que d'éventuels éléments de fixation osseuse (25) sont pourvues d'un revêtement microbicide contenant de préférence des ions argent.

35. Dispositif de fixation osseuse selon l'une quelconque des revendications 1 à 34, **caractérisé en ce que** le clou intramédullaire (1) est réalisé sous une forme asymétrique.

36. Dispositif de fixation osseuse selon l'une quelconque des revendications 1 à 35, **caractérisé en ce que** le clou intramédullaire (1) présente, au niveau de son premier plan (frontal ou latéro-médial), une première courbure et, au niveau de son deuxième plan (sagittal ou antéro-postérieur) perpendiculaire au premier plan, une deuxième courbure.

37. Dispositif de fixation osseuse selon l'une quelconque des revendications 1 à 36, **caractérisé en ce qu'**il comprend en outre un ou plusieurs des éléments suivants :
- une vis de poussée
- une vis de traction
- une vis de compression
- une vis d'arrêt
en tant qu'éléments de fixation osseuse (25).

38. Dispositif de fixation osseuse selon l'une quelconque des revendications 1 à 37, **caractérisé en ce que** le clou intramédullaire (1) présente un canal, de préférence un canal traversant.

39. Dispositif de fixation osseuse selon l'une quelconque des revendications 1 à 38, **caractérisé en ce que** les éléments de fixation osseuse (25) sont réalisés en tant que fils intramédullaires élastiques.

40. Dispositif de fixation osseuse selon l'une quelconque des revendications 1 à 38, **caractérisé en ce que** les éléments de fixation osseuse (25) comprennent des douilles (26) qui sont pressées dans des logements (28) dans la plaque d'ostéosynthèse (10) et dont les alésages centraux permettent d'introduire des vis à os.
